(19) Europäisches Patentamt
European Patent Office
Office européen des brevets

(11) **EP 4 335 468 A1**

(12) **EUROPEAN PATENT APPLICATION**

(43) Date of publication:
**13.03.2024 Bulletin 2024/11**

(21) Application number: **22194710.4**

(22) Date of filing: **08.09.2022**

(51) International Patent Classification (IPC):
**A61M 1/16** *(2006.01)* **A61M 1/28** *(2006.01)*

(52) Cooperative Patent Classification (CPC):
**A61M 1/1696;** A61M 1/287; A61M 2209/088

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fraunhofer-Gesellschaft zur Förderung der angewandten Forschung e.V.**
**80686 München (DE)**

(72) Inventor: **GOLDAU, Rainer**
**04103 Leipzig (DE)**

(74) Representative: **Friese Goeden Patentanwälte PartGmbB**
**Widenmayerstraße 49**
**80538 München (DE)**

(54) **APPARATUS FOR GENERATING DIALYSATE FOR DIALYSIS**

(57) The invention relates to an apparatus for generating dialysate for dialysis comprising dialysate purifying means (300) having a dialysate inlet (301) being adapted and arranged for receiving dialysate to be purified, at least one dialysate concentrating means (310) being adapted and arranged for concentrating substances to be maintained in the dialysate, the dialysate concentrating means (310) having a concentrate outlet (311) and a diluate outlet (312), and a dialysate outlet (302) being adapted and arranged for feeding back the purified dialysate, wherein the concentrate outlet (311) of the at least one dialysate concentrating means (310) is connected to the dialysate outlet (302), further comprising at least one water separating means (400) being adapted and arranged for separating water, the at least one water separating means (400) having an inlet (414) and a water outlet (412), wherein the inlet (414) is connected to the diluate outlet (312) of the at least one dialysate concentrating means (310) and the water outlet (412) of the at least one water separating means (400) is connected to the dialysate outlet (302).

Fig. 1

## Description

The invention relates to an apparatus for generating dialysate for dialysis comprising dialysate purifying means having a dialysate inlet being adapted and arranged for receiving dialysate to be purified, at least one first dialysate purifying means being adapted and arranged for purifying the received dialysate under controlled preservation of particular ingredients like electrolytes and bicarbonate, and a dialysate outlet being adapted and arranged for feeding back the purified dialysate including the ingredients preserved.

## Background of the invention

Patients with severe kidney failure are constrained to continuous dialysis treatment if no matching donor organ can be found or they are in an adverse transplant condition. Without profound medical support this condition is life-threatening and terminal in many cases.

Donor organs are scarce and dialysis is a solution that falls by far short of a healthy kidney as it can rarely be provided continuously and in same quality as from kidney. In most cases patients have to undergo hemodialysis - at least after a few years - often in an intermittent regime 3 by 4 hours per week. Hemodialysis directly accesses the bloodstream and removes uremic toxins. Due to the high physical and mental burden these patients are essentially restricted socially and in their health, joined by high risk of secondary comorbidities and significantly reduced life expectancy. Vast efforts to improve mode and quality of dialysis have been conducted to better integrate it into everyday life.

In particular provision of fresh dialysate itself (min 210 l/week) with its essential ingredients is a problematic issue preventing dialysis from being more continuous or even mobile.

Prior art technical solutions are still not able to adequately reclaim the water and essential solutes discarded during dialysis. Adequacy does not only refer to economic costs for the required quality of the dialysis water and ingredients but also to the fluid volume lost when filters have to discard retentate. The retentate volume is too large to be replenished by the patient's body water.

Furthermore electrolytes and other solutes removed by dialysate reclamation principles cannot be fed back to the patients using prior art techniques and must be balanced and provided from fresh supply within an admixture unit. This makes dialysis costly, susceptible to faults (for instance mixing failures), unsuitable for mobile applications and patients are dependent on local technical and storage facilities.

Continuous, unlimited mobility at any time as with healthy people is achievable for dialysis patients only in rare cases and to a limited extent. There is a need for a solution enabling dialysis patients to freely move like healthy people without considerable restrictions caused by their treatment. Wearable or even implantable solutions fulfilling this requirement are lacking so far.

The amount of water to be cleared by expensive and sometimes unwieldy filter technology prevents this. In order to solve the water problem of hemodialysis EP 3 487 555 B1 proposes to use the cleaning effect of freezing water. Ice crystals exclude any contaminations when they form and 'only' need to be washed externally. The own melting water can be used for this purpose within a wash column. This solution at least can solve the water problem of dialysis by generating fresh water and a drain aliquot concentrated to an extent that permits refilling this volume from the patient's own body water without dehydration.

However, this solution - like all the others listed below - has a considerable shortcoming as it cannot resolve the electrolyte, glucose and bicarbonate loss problem accompanied by todays dialysate reclamation. These substances -sometimes denoted as 'concentrate / bic' in dialysis technology- are removed by all of the prior art approaches. Additional advanced technology is required to reclaim the substances that today are admixed using concentrate / bic to prepare water for dialysis when full mobility and flexibility should be achieved. This technology does not exist so far - but the combination of both would be very desirable in order to approach the up-concentration ability of the native kidney. It would mean to engineer a technical system with an essential core functionality of the nephron, as in terms of reclamation the native kidney does exactly this - it reclaims water from the 180l daily primary urine except the amount of secondary urine discharged via the bladder and it reclaims all useful substances that come with the primary urine.

The water part has been addressed so far by years of development, there are several further prior art suggestions to reclaim dialysis water only, but except one all without reclaiming the solutes:

a.) Absorption of impurities in filter cartridges and ion exchangers.
This solution is expensive and chemically ambitious, especially because carbon adsorbers cannot bind sufficient urea and the ions exchanged must not be harmful or interfere with medical prescriptions.

b.) Use of urease to crack urea, resulting in $NH_3$ and $CO_2$.
This solution has to be combined with solution a. As urease being an enzyme it requires a controlled cooling chain. Its operation must be monitored for efficiency, filter breakthrough and $NH_3$ overload.

c.) Electrosorption and/or reverse osmosis. The large retentate volume required to keep filters open cannot be removed from the patients. Electrosorption also requires biocompatible adsorber surfaces that stand electrical currents without denaturation.

d.) Donor organs from the patient's own stem cells, as well as animal scaffold organs or xenografts recellularized with their differentiated form. There have been intensive research efforts in this field for a long period with even encouraging results, yet still being a scientific domain predominantly - a solution tailored to the patient's everyday needs by far has not yet been accomplished. The complexity of the kidney and vascularization as well as immunological aspects are the main issue to be resolved. These mechanisms do not yet seem fully understood.

e.) Electrochemical detoxification and electrolysis. Here are the same problems as mentioned above in point a. and c.

f.) Electrophoresis. Very high voltages are introduced and the mechanism does not seem to be fully disclosed in document US 2003/187380 A1.

g.) Distillation. This solution consumes much energy and is not reliable enough for urea, urea can pass a distillation column.

h.) Forward osmosis (generation of the osmotic pressure used to extract water from the waste dialysate by solutes that can be easily filtered out later by reverse osmosis). All osmosis procedures require a constant flow of water, which is lost in parts in single-stage procedures (retentate).
The procedures require too much water replacement for the patients, which they cannot provide by drinking. Remaining solutes could be harmful, and the cost of providing them in forward osmosis can be high.

i.) Advanced approaches exist conducted by a research group labeled 'The kidney project' to generate a primary filtrate directly from blood by means of ultrafiltration slits engraved in semiconductors, dimensioned to mimic the primary filter slits of podocytes present within a glomerulus. This primary urine is conducted into a bioreactor containing tubule cells, which are expected to extract and separate uremic toxins and drain them to the bladder. Primary urine is also called primitive urine. In this disclosure the term primary urine will be used for primary or primitive urine. The substances to be recovered however are transferred back into the blood system via a second semiconductor slit system populated with tubular cells that are separated from the immunoresponse of the body this way. Thus, this approach would even address both partial problems - recover water as well as essential substances that are selected by the cells. Both functional subunits are intended to be combined and implanted as a full nephron replacement system. With this combined system even further functionality from the hormonal field shall be realized, so that the body can control its diuresis with the system via ADH, for example. This approach can be regarded as very sophisticated in comparison to technical solutions available today. This solution provides many cells on a large surface area, each of which transports water and electrolytes from the bioreactor (primary urinary compartment) into the serum via a low osmotic gradient. In this way, however, concentration to osmolarities exceeding the serum about fivefold, as in urine, will be impossible. To keep homoeostasis the patients may only discard a certain amount of water, which is small compared to the primary urine volume. How the resulting high water loss shall be compensated has not been laid open so far. Further it is not apparent how the bioreactor can apply the fundamental principle of the kidney to concentrate urine, i.e. the countercurrent multiplication principle (CMP) and reduce fluid loss that way. So in a synoptic summary this solution is very encouraging but lacking at least this essential functionality.

j.) EP 2 586 473 A1 proposes to use a sequence of glomerulus, tubule and loop of Henle in the form of components connected by wires - realized by semiconductor technology (nephron on a chip). The sequence is operated with blood and water and seems to be functional only as a whole - no individual component has attributed an independent technical feasibility. After filling up blood at the artificial glomeruli, the larger-molecular components are left in the blood, the smaller ones (glucose, urea) are removed and directed to the next stage, the tubules. There, a separation of smaller-molecular substances (glucose from urea) is carried out. This happens purely diffusively, without CMP and without using any hydraulic pressure. Separation stages for different molecular sizes are used. In the third stage, the loop, the water balance is controlled. Here a semipermeable membrane is used and the functional principle is explained as diffusion. However CMP is not mentioned or suggested. Instead, the document is teaching away from CMP, because in the figures the flow direction arrows are arranged differently than would be required for the CMP. It is only generally referred to osmosis being effective to separate urea and water, whereas this is impossible due to very similar molecular size. In particular, no reference is made to the flow conditions necessary to achieve a multiplying effect (cross-sections, counterflow, residence time and contact time). It is not clear from the figures that a counterflow is used. Furthermore, the document is silent with respect to the fluid balance. The prior art document is rather a general and not completely accurate representation of the nephron function, which is also divided into sub-functions other than the sequence of stages according to molecule size because Henle's loop is open for any molecular size. This document neither discloses nor suggests using CMP and thus cannot upconcentrate primary urine.

k.) E. WEINBERG et al, "Concept and computational design for a bioartificial nephron-on-a-chip", The international journal of artificial organs, Vol.31, No. 6, pages 508-514, June 2008 discloses to use CMP for the simulation of the nephron function. There, the use of countercurrent flow, small conduction cross-sections and low longitudinal diffusion is proposed, with fluid back-transfer into blood vessels built directly into the arrangement. This prior art solution has the disadvantage that the blood vessels to be provided must have a diameter being only slightly thicker than the cell diameter in order to achieve the necessary low longitudinal diffusion and to divide water volume elements into sufficiently small sections. With the technology currently available, it is not possible to provide artificial blood vessels that are thin enough to take over the functionality of the vasa recta in CMP. The hollow fibers used in dialysers are about 20-30 times larger in diameter and even with such big diameters there are occasional difficulties due to occlusion. Blood is not avoided here but avoiding blood vessels and cells in a technical setup is one major advantage of the present invention. Further it will become clear that the use of technical membranes instead of cells allows to fractionate different molecular subgroups solved in the primary urine in a controlled manner.

There are numerous different combinations of the principles listed above, however none of them is able to accomplish both aspects simultaneously like the kidney: To reclaim water and to recover all essential substances that must not be discarded or may only be discarded in small amounts. So far todays status of dialysis water reclamation is not yet satisfying.

Accordingly, it is an object of the present invention to provide an apparatus for generating dialysate for dialysis being able to reclaim water as well as to recover all essential substances that must not be discarded or may only be discarded in small amounts.

## Summary of the invention

The object of the invention is achieved by an apparatus for generating dialysate for dialysis with the features of claim 1. Preferred embodiments of the invention are disclosed in the dependent claims.

In accordance with the present invention there is provided an apparatus for generating dialysate for dialysis comprising dialysate purifying means having a dialysate inlet being adapted and arranged for receiving dialysate to be purified, at least one dialysate concentrating means being adapted and arranged for concentrating substances to be maintained in the dialysate, the dialysate concentrating means having a concentrate outlet and a diluate outlet, and a dialysate outlet being adapted and arranged for feeding back the purified dialysate, wherein the concentrate outlet of the at least one dialysate concentrating means is connected to the dialysate outlet, the apparatus for generating dialysate for dialysis further comprising at least one water separating means being adapted and arranged for separating water, the at least one water separating means having an inlet and a water outlet, wherein the inlet of the at least one water separating means is connected to the diluate outlet of the at least one dialysate concentrating means and the water outlet of the at least one water separating means is connected to the dialysate outlet.

The apparatus in accordance with the invention has the advantage that the dialysate and/or diluate can be reclaimed by recirculating a portion of the dialysate and/or diluate from the inlet to the outlet, wherein another portion thereof is conducted to a drain. The apparatus in accordance with the invention can be used with any type of medical dialysis comprising dialysate concentrating means and/or dialysate purifying means used as single elementary component or aggregated in higher numbers in one or several concentrating and/or purifying stages in combination with the water separating means such that the water can be recirculated.

The present invention is substantially different from all principles mentioned above in a.) - k.). It simultaneously accomplishes to reclaim water and purify it from also urea without discarding more water than patients could replace from their body water, it concentrates the secondary urine to physiological levels like the kidney does, it recovers or maintains electrolytes and small substances of the primary urine in a controlled and sufficient manner, is able to separate different fractions of molecular size from the primary urine e.g. to improve transport protein discharge and can be downsized to portable base station and wearable dialysis vest solutions for continuous dialysis at home, at work or on vacation.

It further has potential to be combined with solution i.) in a final fully engineered and elaborated version and may completely be wearable. It is not expected to be implantable soon as disposable filters will still play a major role for a long time. But even implanting the device of the invention can be considered once filter technology has advanced significantly.

According to the invention the apparatus may have at least one dialysate purifying means being adapted and arranged for removing substances from the dialysate, the dialysate purifying means having a concentrate outlet and a diluate outlet, wherein the diluate outlet of the at least one dialysate purifying means is connected to the dialysate outlet.

According to the invention the parameters solute osmolarity, size, pressure, membrane type, permeability, thickness and temperature of the dialysate concentrating means and/or dialysate purifying means may differ in the separate stages.

According to the invention the apparatus may have a dialysate or diluate inlet from any primary fluid generation device that may be connected to the human blood circuit or equivalents, and may have a dialysate outlet or dialysate return that returns the purified fresh dialysate or diluate containing a biocompatible amount of electrolytes and bicarbonate to the blood directly or to the fluid generation device.

According to the invention each of the dialysate concentrating means and/or dialysate purifying means and/or water separating means may have a common outlet to drain. Alternatively or in addition one or more means may have a separate outlet to drain.

According to the invention the dialysate concentrating means and/or dialysate purifying means and/or water separating means may be connected in a manner that the concentrate outlet of a dialysate concentrating means is connected to the inlet of one or more of the following dialysate concentrating means and/or dialysate purifying means and/or water separating means. Alternatively or in addition the outlet of one or more dialysate concentrating means and/or dialysate purifying means and/or water separating means may be connected in a manner that the depleted outlet of the means is connected to the inlet to one or more of the following means whereas the connection scheme implemented is governed by achievement of convenient fluid combination of the different units that match the medical prescription for return and removal of water, solutes and uremic toxins or fractions of uremic toxins.

According to the invention the apparatus may further comprise one or more pumps for the transport of fluid and for generation of hydraulic pressure as well as regulation valves to control the different flows in the flow lines.

The advantages of the apparatus in accordance with the present invention may also be summarized as follows: In 1951 Hargitay published a paper (Hargitay1951) - which is referenced and discussed later in detail - with a mathematical analysis resulting in the fundamental differential equation of the kidneys up-concentration mechanism that is based on the countercurrent multiplication principle (CMP), further an experimental proof of his theory based on a sodium salt but not on urea. The principle explains mathematically how kidneys can produce extreme concentrations. Today his theory is widespread accepted although not applied to the technical world because ion pumps are regarded to be missing as well as urea separation means. The invention does not use ion pumps but uses hydraulic pressure instead and relates to the use of this principle for chemical and pharmaceutical dialysis based purification processes wherever small amounts of high concentrate are needed as well as to medical dialysis, where it can be used to clear dialysate - the wash fluid used for dialysis. It could produce artificial urine without discarding too much electrolytes and bicarbonate and reclaiming the water if there was no urea also. The technical extraction of urea from dialysate -predominant in medical dialysis- still is unresolved using membrane based technology lacking urea- or water-specific transporters that exist in the kidney but do not technically at a satisfying elaboration level. The invention combines the kidney based countercurrent multiplication principle originating from Hargitay1951 with a cryo-purification principle that is capable to remove urea from water by forming ice crystals that are rinsed with their own melt water in a wash column known from prior art in the beverage industries. The combination of both principles countercurrent multiplication and cryo-purification allows to reclaim primary urine once won from prior art filters and discard a convenient amount of secondary urine. The invention limits water loss, electrolyte and bicarbonate loss to values similar to the loss the native kidney mediates when producing secondary urine. Thus the invention is a kind of technical kidney that covers the up-concentration and removal function of the human kidney also for urea and can be used to source a wearable artificial kidney system with dialysate without need for additional external support except electricity. The invention comprises a wearable dialysis system based on the combination of the two principles that allows mobility and full social and economic participation to the patients.

According to the invention the at least one water separating means can comprise a cryo-purifier.

According to the invention the at least one water separating means can comprise means for removing urea from aqueous solutions and/or urease means and/or at least one aquaporin membrane.

These embodiments have the advantage, that water for resupplying to the patient can be obtained by the water separating means.

According to the invention the cryo-purifier can comprise an ice crystal growth container and/or a wash column.

According to the invention the at least one dialysate concentrating means and/or the at least one dialysate purifying means can be adapted and arranged according to the CMP.

According to the invention the cryo-purifier or cryo-purification unit may be replaced by any other type of device capable of removing urea from aqueous solutions. The skilled person is aware of suitable urease based solutions or devices based on the use of aquaporin membranes. It is expected that these membranes may be improved in the future such that these membranes may provide a sufficient separation between urea and water especially when used in an installation using the countercurrent multiplication principle.

According to the invention the at least one dialysate concentrating means and/or the at least one dialysate purifying means can comprise a semipermeable filter means, a first flow path at a first side of the semipermeable filter means, and a second flow path at a second side of the semipermeable filter means, being opposite to the first side of the semipermeable filter means.

According to the invention the at least one dialysate concentrating means and/or the at least one dialysate purifying means can comprise pump means being adapted and arranged for applying pressure to the at least one dialysate concentrating means and/or the at least one dialysate purifying means.

According to the invention the at least one dialysate concentrating means and/or the at least one dialysate purifying means can be adapted and arranged to provide several successive and/or concurrent stages.

According to the invention the at least one dialysate concentrating means can be adapted and arranged to provide at least one cutoff for electrolytes and/or bicarbonates.

According to the invention the at least one dialysate purifying means can be adapted and arranged to provide at least one cutoff for uremic toxins.

According to the invention the apparatus can comprise several dialysate purifying means. The separate dialysate purifying means may have with different types of semipermeable membranes in their elementary countercurrent multiplication principle device so a fraction of the smallest, a midsize range or larger size solute molecules or uremic toxins with a fraction of solvent can be separated from the depleted or concentrated outlets and separately be returned to the dialysate outlet of the apparatus. The fractions may be submitted to special treatment or be discarded to drain comprising means that regulate the amount of fluid directed to the flow lines that a desired prescription can be matched.

According to the invention the apparatus can further comprise at least one additional dialysate purifying means being adapted and arranged to provide at least one cutoff for transport proteins. Thereby the at least one additional dialysate purifying means can preferably comprise heating means. Alternatively and/or in addition the at least one additional dialysate purifying means can preferably comprise pH modification means.

According to the invention, the heating means can be preferably located close to the semipermeable membrane of the additional dialysate purifying means.

According to the invention, the apparatus may comprise a fluid generation unit that has elevated cutoff compared to regular hemodialysis to allow transport proteins from serum to pass the membrane to a certain extent, thus superimposing slight plasmapheresis to hemodialysis.

According to the invention, the apparatus can further comprise a separation stage for highest molecular weight fraction which includes a heating or a pH modification means close to the semipermeable membrane that heats the transport proteins up to highest non-denaturation temperature that will enhance the chance for unloading them or a pH that also unloads them to a certain extent. According to the invention this may occur in combination with the hydraulic pressure that drives the CMP principle to drag the load released across the membrane, which is designed to let the load pass through the pores, but not the transport proteins, further returning the unloaded transport proteins via the dialysate outlet and the dialysate filter to the body or discarding them to a desired extent.

According to the invention, the apparatus can further comprise a fluid generation unit for producing primary urine. The fluid generation unit may be based on semiconductors. In addition or alternatively the fluid generation unit may be based on ceramics. In addition or alternatively the fluid generation unit may be based on hollow fibers. The hollow fibers may be durable hollow fibers.

According to the invention, the dialysate inlet and the dialysate outlet may be connected to the fluid generation unit.

According to the invention, the apparatus may be enclosed in a transportable housing. This has the advantage that the apparatus can be deposited at work, at home, in a car or like a trolley on travel acting like a base station for dialysis fluid provision which is permanently or intermittently attached to a wearable dialysis device e.g. in a vest, trousers or belt to provide the wearable part with fresh dialysis water at desired temperature and to receive the waste water. The apparatus can be adapted and arranged to provide energy and/or data to the wearable part of the apparatus. Alternatively the wearable part has its own energy source and/or data source. According to the invention, the wearable part of the apparatus can perform dialysis to the patient even in periods when the wearable part is not connected to the base station of the apparatus. This enables the apparatus to be used for ambulant continuous hemo- or peritoneal dialysis independent of the base-station while the wearable part may not be attached and further may or may not hold remote connection to medical surveillance.

The present invention is not limited to patient dialysis only, but can advantageously also be used for dialysis in the former, broader sense to separate solutes into only one out of any two or more fluids.

According to the invention the apparatus can be implanted into the human body whereas all internal means and filters are made of ceramics and/or semiconductors and/or (durable) hollow fibers. This has the advantage, that there is no need for replacement. All blood connections can be made internally and the outlet may be connected to the ureter. Electrical energy for hydraulic pumps and valves can be supplied from extern and/or and internal energy source.

In accordance with the present invention there is also provided a hydraulic pressure driven solute concentrating means being adapted and arranged for concentrating substances to be maintained as solute in the solvent where the solute is solved, the solute concentrating means having a concentrate outlet and a diluate outlet, and a first semipermeable filter means being adapted and arranged to separate first solutes from the solvent, the semipermeable filter means being located between a first flow path at a first side of the semipermeable filter means, and a second flow path at a second side of the semipermeable filter means, being opposite to the first side of the semipermeable filter means, wherein the first flow path and the second flow path are adapted and arranged to have at least one cross section wherein the first flow path surrounds the second flow path or the second flow path surrounds the first flow path.

In accordance with the present invention there is also provided an solute concentrating means being adapted and arranged for concentrating substances to be maintained as solute in the solvent where the solute is solved, the solute concentrating means having a concentrate outlet and a diluate outlet, and a first semipermeable filter means being adapted and arranged to separate first solutes from the solvent, the semipermeable filter means being located between a first flow path at a first side of the semipermeable filter means, and a second flow path at a second side of the semipermeable filter means, being opposite to the first side of the semipermeable filter means, wherein the first flow path and the second flow path are adapted and arranged to have at least one cross section wherein the first flow path surrounds the second flow path or the second flow path surrounds the first flow path.

In accordance with the present invention the semipermeable filter means may be adapted and arranged to have a tubular form.

In accordance with the present invention the semipermeable filter means may be adapted and arranged to have a tubular form having a diameter changing along the course of the first and second flow path.

In accordance with the present invention the semipermeable filter means may be adapted and arranged to have a tubular form having an almost constant or constant diameter along the course of the first and second flow path.

In accordance with the present invention the first flow path and second flow path may be arranged and adapted to be concentric.

In accordance with the present invention the first flow path may be arranged inside the tubular form of the semipermeable filter means and the second flow path may be arranged outside the tubular form of the semipermeable filter means.

In accordance with the present invention the second flow path may be arranged inside the tubular form of the semipermeable filter means and the first flow path may be arranged outside the tubular form of the semipermeable filter means.

In accordance with the present invention the solute concentrating means may further comprise second semipermeable filter means.

In accordance with the present invention the second semipermeable filter means may be adapted and arranged to separate a third flow path from the first flow path.

In accordance with the present invention the second semipermeable filter means may be adapted and arranged to separate a third flow path from the second flow path.

In accordance with the present invention the second semipermeable filter means may be adapted and arranged to separate a third flow path from a flow path connected to the output of the first flow path.

In accordance with the present invention the second semipermeable filter means may be adapted and arranged to separate a third flow path from a flow path connected to the output of the second flow path.

In accordance with the present invention the second semipermeable filter means may be adapted and arranged to surround the first semipermeable filter means. Thereby the second semipermeable filter means may be arranged between the third flow path and the second flow path. Further, the first semipermeable filter means may be arranged between the second flow path and the first flow path.

In accordance with the present invention the second semipermeable filter means and the first semipermeable filter means may be adapted and arranged in the same housing and/or the same tube.

In accordance with the present invention the second semipermeable filter means and the first semipermeable filter means may be adapted and arranged in a single housing and/or single tube.

In accordance with the present invention the second semipermeable filter means and the first semipermeable filter means may be adapted and arranged in separate housings and/or tubes.

In accordance with the present invention there is also provided a hydraulic pressure driven solute concentration apparatus comprising a fluid inlet for osmotic active fluid of type 1 and/or type 2, an fluid outlet and a collective duct inlet for osmotic active inlet fluid type 1 and/or type 2, further comprising a semipermeable membrane of first type to separate solutes or a solute group of type 1 from the solvent, the semipermeable membrane being located between a descending limb and an ascending limb.

According to the invention the hydraulic pressure driven solute concentrating means can further comprise a collective duct and a second semipermeable filter means being adapted and arranged to separate second solutes from the solvent, the second semipermeable filter means being located between the second flow path and the collective duct for separating the second flow path and the collective duct.

According to the invention the hydraulic pressure driven solute concentrating means further can comprise a collective duct.

According to the invention the hydraulic pressure driven solute concentrating means further can comprise a further semipermeable membrane.

According to the invention the first semipermeable filter means and the second semipermeable filter means can be adapted and arranged to separate different solute types.

According to the invention the first semipermeable filter means and the second semipermeable filter means can be are adapted and arranged to separate the same or similar different solute types.

According to the invention the hydraulic pressure driven solute concentrating means can comprise a second type of solutes or solute group of type 2 to be separated from the solvent.

According to the invention the hydraulic pressure driven solute concentrating means can comprise a first solvent in the descending limb and the and ascending limb. According to the invention the hydraulic pressure driven solute concentrating means can comprise the same solvent in the collective duct. Alternatively the hydraulic pressure driven solute concentrating means can comprise the second solvent in the collective duct being different than the first solvent.

According to the invention the hydraulic pressure driven solute concentrating means can comprise one concentrate outlet or several concentrate outlets at an appropriate point along the first flow path and/or the second flow path and/or the collective duct.

According to the invention the hydraulic pressure driven solute concentrating means can comprise one concentrate outlet or several concentrate outlets at an appropriate point along the descending limb and/or the ascending limb and/or the collective duct.

According to the invention the hydraulic pressure driven solute concentrating means can be operated at parameters of duct cross-section, shape, arrangement, length, membrane permeability, pressure, pressure profile, osmolarity and flow speed being compatible to the countercurrent multiplication principle to be effective.

According to the invention the hydraulic pressure driven solute concentrating means can comprise a hairpin bend being adapted and arranged to connect the first flow path with the second flow path.

According to the invention the hydraulic pressure driven solute concentrating means can comprise a hairpin bend being adapted and arranged to connect the descending limb with the ascending limb.

According to the invention the hairpin bend can be adapted and arranged to connect the collective duct with the second flow path.

According to the invention the hairpin bend can be adapted and arranged to connect the collective duct with the ascending limb.

According to the invention the hydraulic pressure driven solute concentrating means can comprise a pressure drop valve at the hairpin bend.

According to the invention the first semipermeable filter means and the second semipermeable filter means can be adapted and arranged in the same housing and/or module.

According to the invention the first semipermeable filter means and the second semipermeable filter means can be adapted and arranged in separate housings and/or modules.

According to the invention the hydraulic pressure driven solute concentrating means can comprise a regulated valve being adapted and arranged to regulate the inlet flow.

According to the invention the hydraulic pressure driven solute concentrating means can comprise a regulated valve being adapted and arranged to regulate the outlet flow.

According to the invention the hydraulic pressure driven solute concentrating means can be adapted and arranged such any of the outlets are operating with different osmolarity or composition than any inlet which may be used for further process steps of chemical, pharmaceutical or physical nature.

According to the invention the hydraulic pressure driven solute concentrating means can be adapted and arranged such any of the outlets can be discarded.

According to the invention the hydraulic pressure driven solute concentrating means can be adapted and arranged such any of the outlets can be maintained for purification or separation purposes.

According to the invention the hydraulic pressure driven solute concentrating means can be used as single unit.

According to the invention the hydraulic pressure driven solute concentrating means can be used in parallel to further hydraulic pressure driven solute concentrating means.

According to the invention the hydraulic pressure driven solute concentrating means can be used in series to further hydraulic pressure driven solute concentrating means.

According to the invention the hydraulic pressure driven solute concentrating means can be used in series and in parallel to further hydraulic pressure driven solute concentrating means.

According to the invention the hydraulic pressure driven solute concentrating means can be used simultaneously to further hydraulic pressure driven solute concentrating means.

According to the invention the hydraulic pressure driven solute concentrating means can be used in time shifted operation to further hydraulic pressure driven solute concentrating means.

According to the invention the hydraulic pressure driven solute concentrating means can be used in a spatially cumulated arrangement to further hydraulic pressure driven solute concentrating means.

According to the invention the hydraulic pressure driven solute concentrating means can be used in a spatially distributed arrangement to further hydraulic pressure driven solute concentrating means.

According to the invention the hydraulic pressure driven solute concentrating means can be adapted and arranged like the tubules and/or the Henle's loops and/or the collective ducts in the native kidney, preferably including the generation of localized regions with high osmolarity.

According to the invention the hydraulic pressure driven solute concentrating means can comprise an inlet with a controlled valve adapted and arranged for controlling the pressure at the inlet.

According to the invention the collective duct of the hydraulic pressure driven solute concentrating means can comprise an inlet with a controlled valve adapted and arranged for controlling the pressure at the inlet of the collective duct.

According to the invention the at least one dialysate concentrating means and/or several dialysate concentrating means can be adapted and arranged according to a hydraulic pressure driven solute concentrating means as detailed above and in this disclosure.

The present invention is not limited to hemodialysis, i.e. there are many technical areas where pressure-based concentration without significant solvent loss is of great importance. This is especially the case with expensive solvents to reduce solvent loss, as it is for water in the case of medical dialysis.

The invention is described with reference to the figures showing embodiments of the invention, wherein the following reference signs will be used:

| 1 | wearable part |
|---|---|
| 2 | connection lines |
| 3 | portable part |
| 4 | discharge means |
| 101 | inlet |
| 102 | inlet nozzle |
| 103 | Crystallizer-heater-cooler unit |
| 104 | Seed crystal inlet nozzle |
| 105 | Container for ice crystal growth |
| 106 | cooling jacket of 105 |
| 107 | ice slurry |
| 108 | stirrer |
| 109 | feedback for seed preparation |
| 110 | slurry feed for wash column |
| 111 | slurry inlet to wash column |
| 112 | wash column (wc) |
| 113 | piston to push slurry through wc 12 |
| 114 | valves for flow control |
| 115 | liquid phase filter |
| 116 | concentrate outlet |
| 117 | Mixture of liquid and solid phase |
| 118 | wash front |
| 119 | pure ice and pure liquid |
| 120 | rotating ice scraper |
| 121 | heater |
| 122 | pure slurry (solid and liquid) pipe |
| 123 | pump |
| 124 | water (liquid) feedback loop |
| 125 | water outlet for dialysis |
| 126 | pure slurry (solid and liquid) |
| 130 | cryopurifier |
| 300 | apparatus for generating dialysate for dialysis |
| 301 | dialysate inflow line / inlet of 300 |
| 302 | dialysate return line / outlet of 300 |
| 303 | feed back |
| 310 | solute concentrating means / dialysate concentrating means / CMP based separation unit |
| 310A | first stage of 310 |
| 310B | second stage of 310 |

| | |
|---|---|
| 310C | third stage of 310 |
| 310D | fourth stage of 310 |
| 311 | return line /concentrate of 310 |
| 311A | return line /concentrate of 310A |
| 311B | return line /concentrate of 310B |
| 311C | return line /concentrate of 310C |
| 311D | return line /concentrate of 310D |
| 312 | flow line /diluate or 310 |
| 312A | flow line /diluate of 310A |
| 312B | flow line /diluate of 310B |
| 312C | flow line /diluate of 310C |
| 312D | flow line /diluate of 310D |
| 320 | semipermeable filter means |
| 321 | semipermeable membrane |
| 323 | first side of 320 |
| 324 | second side of 320 |
| 325A | arrow (length showing amount of water/solute transport through 321) |
| 325B | arrow (length showing amount of water/solute transport through 321) |
| 325C | arrow (length showing amount of water/solute transport through 321) |
| 325D | arrow (length showing amount of water/solute transport through |
| 321) 330 | tube / pipe |
| 331 | first end of 330 |
| 332 | second end of 330 |
| 333 | first flow path / AL |
| 334 | second flow path / DL |
| 335 | valve |
| 336 | collective duct /CD |
| 337 | outlet orifice |
| 338 | valve |
| 340 | semipermeable filter means |
| 341 | semipermeable membrane |
| 343 | first side of 320 |
| 344 | second side of 320 |
| 345A | arrow (length showing amount of water/solute transport through 341) |
| 345B | arrow (length showing amount of water/solute transport through 341) |
| 345C | arrow (length showing amount of water/solute transport through 341) |
| 345D | arrow (length showing amount of water/solute transport through 341) |
| 350 | tube / pipe |
| 351 | first end of 350 |
| 352 | second end of 350 |
| 353 | first flow path / AL |
| 354 | second flow path / DL |
| 355 | valve |
| 356 | valve |
| 357 | valve |
| 400 | water-urea separating means |
| 410 | cryo-purifier / cryo-based dialysate regeneration unit (or any other unit capable of separating urea from water/solute) |
| 411 | drain line |
| 412 | return line / water return line |
| 413 | final urine outlet line |
| 414 | inlet of 410 |
| 415 | valve |
| 500 | primary fluid generation device (for instance primary urine) |
| 501 | inlet arterial bloodline |
| 502 | venous bloodline |
| 600 | pump means |
| 610 | pump |

| 611 | valve |
| 620 | pump |
| 621 | valve |
| 700 | solute purifying means / dialysate purifying means / CMP based separation unit |
| 700A | solute purifying means / dialysate purifying means / CMP based separation unit |
| 700B | solute purifying means / dialysate purifying means / CMP based separation unit |
| 700C | solute purifying means / dialysate purifying means / CMP based separation unit |
| 701 | concentrate outlet / drain line of 700 |
| 701A | concentrate outlet / drain line of 700A |
| 701B | concentrate outlet / drain line of 700B |
| 701B | concentrate outlet / drain line of 700C |
| 702 | diluate outlet / return line of 700 |
| 702A | diluate outlet / return line of 700A |
| 702B | diluate outlet / return line of 700B |
| 702C | diluate outlet / return line of 700C |
| 710 | valve |
| 720 | valve |
| 730 | valve |
| 740 | valve |
| 750 | valve |
| 760 | valve |
| 770 | valve |
| 780 | heating means |
| 790 | pH modification means |
| 800 | priming amount means |
| AL | ascending limb |
| CD | collective duct |
| CMP | countercurrent multiplication principle |
| DL | descending limb |
| SMP | semipermeable membrane |

**Brief description of the figures**

Fig. 1    is a schematic diagram of an apparatus for generating dialysate for dialysis according to an embodiment of the invention.

Fig. 2    is a schematic diagram of an apparatus for generating dialysate for dialysis according to an embodiment of the invention including multiple separation stages.

Fig. 3    is a schematic diagram of an dialysate purifying means 700 of an apparatus for generating dialysate for dialysis according to an embodiment of the invention, for instance as shown in Fig. 1 or Fig. 2.

Fig. 4    is a schematic diagram of an dialysate purifying means 700B of an apparatus for generating dialysate for dialysis according to an embodiment of the invention, for instance as shown in Fig. 1 or Fig. 2.

Fig. 5    is a schematic diagram of a hydraulic pressure driven solute concentrating means and/or an dialysate concentrating means of an apparatus for generating dialysate for dialysis according to an embodiment of the invention, for instance as shown in Fig. 1 or Fig. 2.

Fig. 6    is a schematic diagram of a hydraulic pressure driven solute concentrating means and/or an dialysate concentrating means of an apparatus for generating dialysate for dialysis according to an embodiment of the invention, for instance as shown in Fig. 1 or Fig. 2.

Fig. 7    is a schematic diagram of a water separating means of an apparatus for generating dialysate for dialysis according to an embodiment of the invention, for instance as shown in Fig. 1 or Fig. 2.

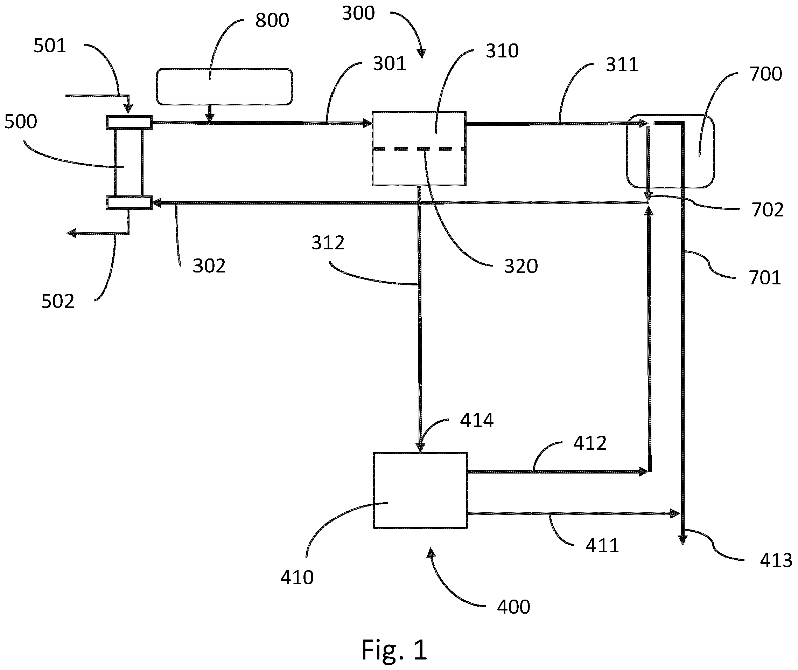

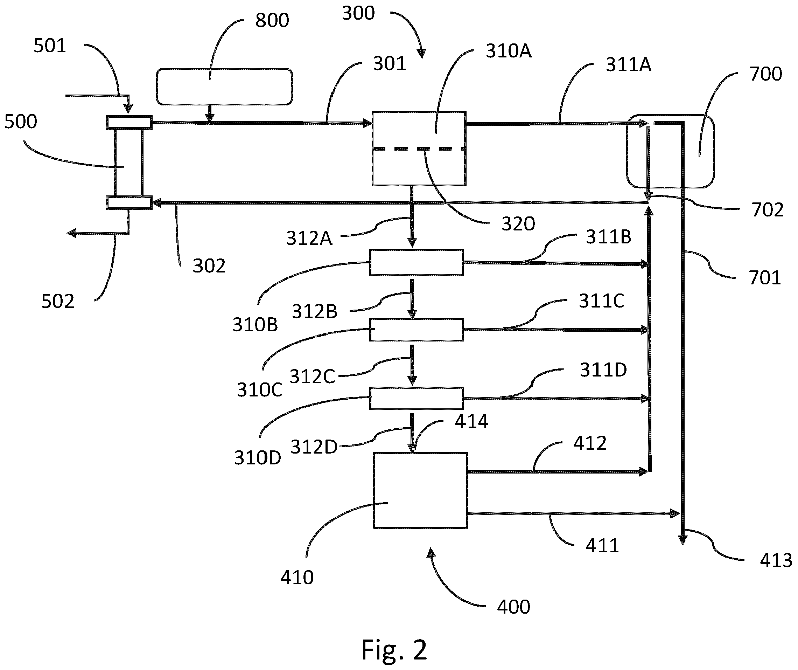

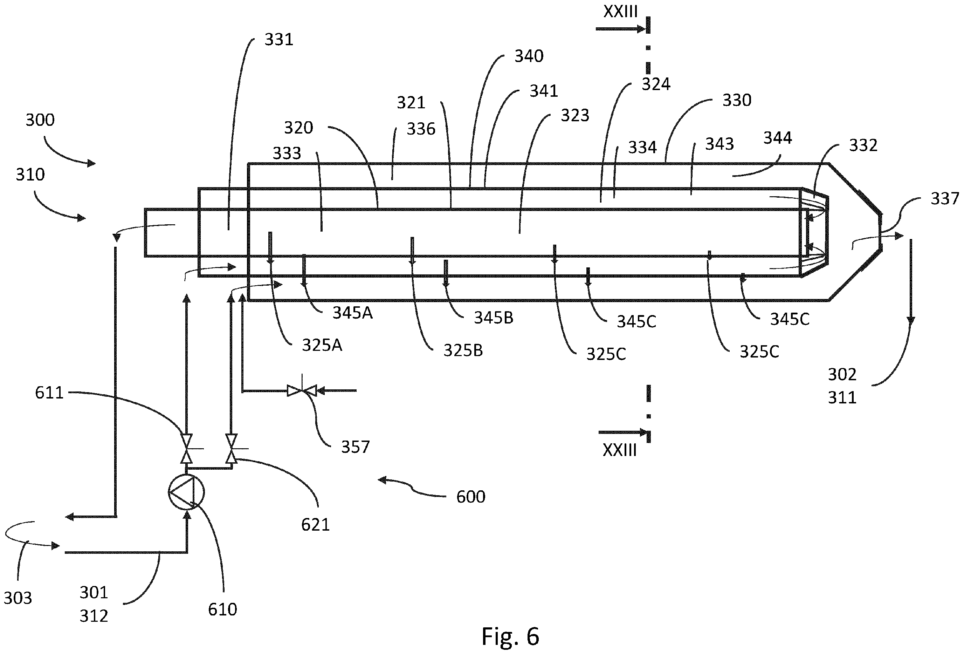

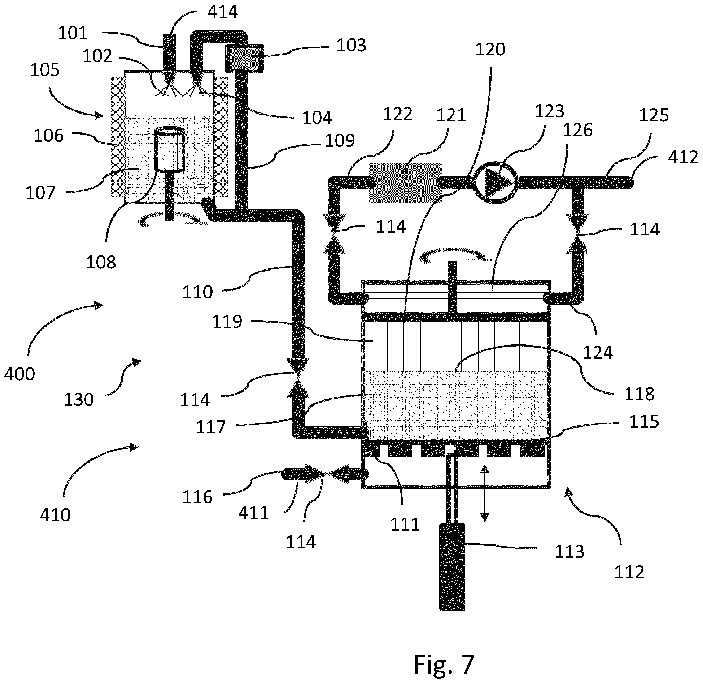

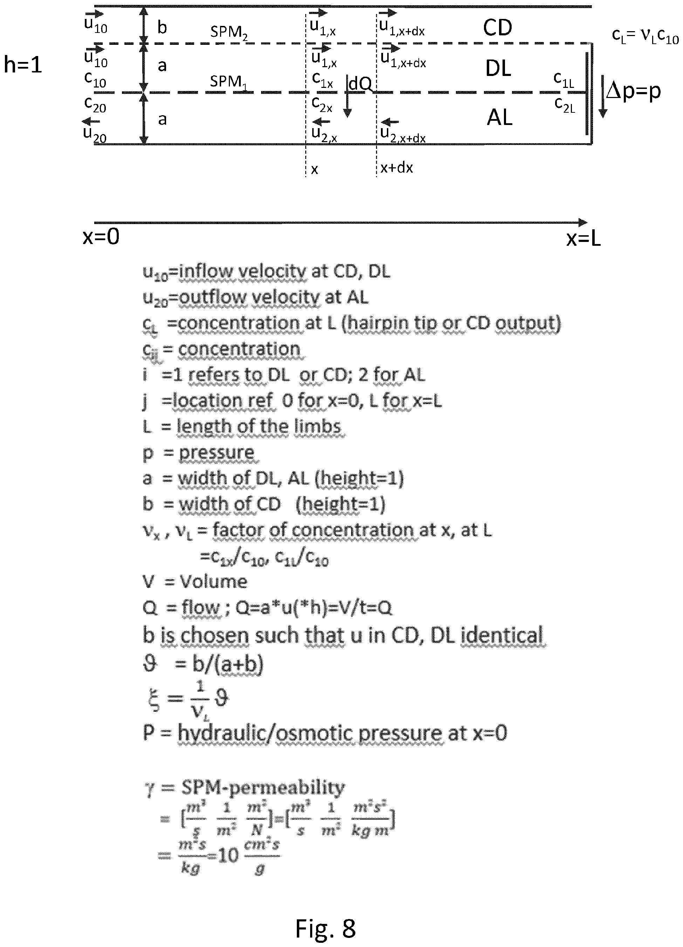

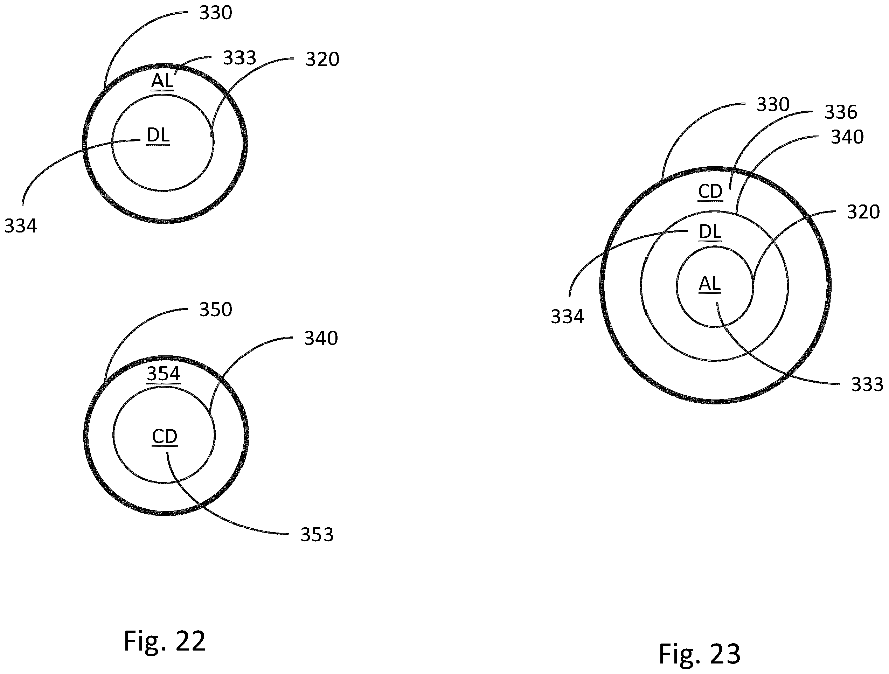

Fig. 8    is a schematic diagram similar to figure 7 of Hargitay1951 which is illustrating the internal structure used for the countercurrent multiplication principle in a hydraulic pressure driven solute concentrating means and/or an dialysate concentrating means of an apparatus for generating dialysate for dialysis according to an embodiment of the invention, for instance as shown in Fig. 1 or Fig. 2 having an collective duct, an ascending limb and a descending limb, and/or for instance as shown in Figs. 6 and 23 wherein only the upper half of the concentric arrangement of the collective duct, the ascending limb and the descending limb is shown in Fig. 8.

Fig. 9    is a schematic diagram showing a finite segment of the internal structure of Fig. 8 and the effect of the pressure on a semipermeable membrane as used in a hydraulic pressure driven solute concentrating means and/or an dialysate concentrating means of an apparatus for generating dialysate for dialysis according to an embodiment of the invention, for instance as shown in Fig. 1 or Fig. 2.

Fig. 10    is a schematic diagram showing steps 1 to 3 of a thought experiment illustrating the operation of a hydraulic pressure driven solute concentrating means and/or an dialysate concentrating means of an apparatus for generating dialysate for dialysis according to an embodiment of the invention, for instance as shown in Fig. 1 or Fig. 2, wherein several finite segments of Fig. 9 are put aside to each other.

Fig. 11    is a schematic diagram showing steps 4 to 6 of the thought experiment of Fig. 10.

Fig. 12    is a schematic diagram showing steps 7 to 8 of the thought experiment of Fig. 10.

Fig. 13    is a diagram showing the concentration and flow velocity versus the length of an analytical model for the thought experiment of Fig. 10 based on the theory of Hargitay1951.

Fig. 14    is a diagram showing the result of Hargitay1951 for solutes that can be separated by a semipermeable membrane..

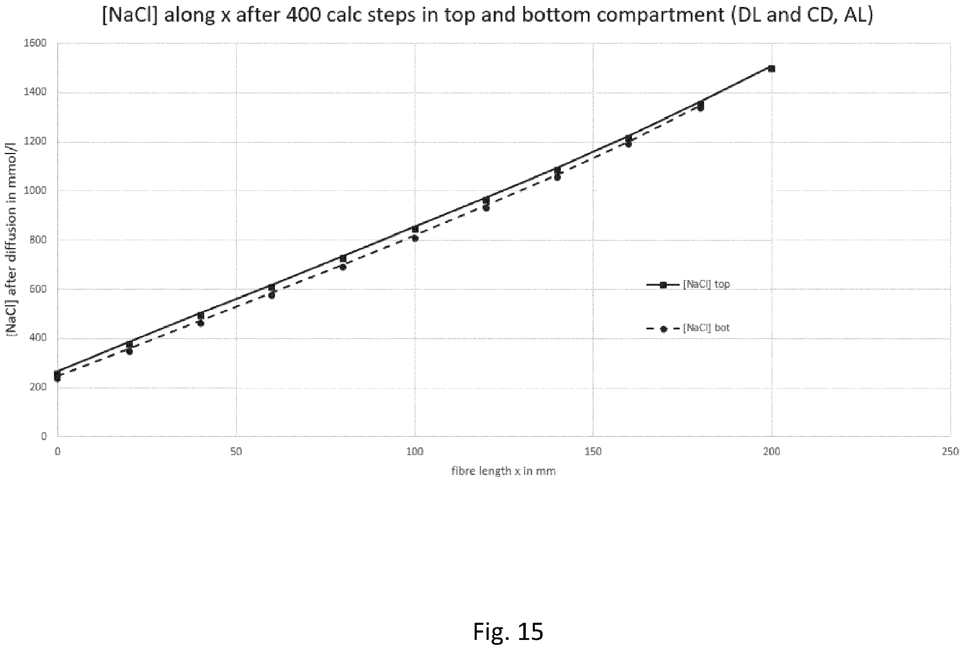

Fig. 15    is a diagram showing the concentration of NaCl at the end of diffusion time of a numerical model for the thought experiment of Fig. 10.

Fig. 16    is a schematic diagram of an apparatus for generating dialysate for dialysis according to an embodiment of the invention that can separate different molecular weight fractions of toxins.

Fig. 17    is a diagram showing the primary urine volume and filter time required versus the # of implemented runs for the thought experiment of Fig. 10.

Fig. 18    is a diagram showing the exchange surface and technical device volume (cumulative) versus the exchange surface [m2] and the volume of the technical device [m3] for the thought experiment of Fig. 10.

Fig. 19    is a diagram showing the clearance of NaCl and urea versus the # of run for the thought experiment of Fig. 10.

Fig. 20    is a schematic view of a portable and wearable unit according to an embodiment of the invention.

Fig. 21    is a schematic diagram of an apparatus for generating dialysate for dialysis according to an embodiment of the invention without optional purifying means.

Fig. 22    is a cross-section of the hydraulic pressure driven solute concentrating means and/or an dialysate concentrating means of an apparatus for generating dialysate for dialysis of Fig. 5 along lines XXII-XXII of Fig. 5.

Fig. 23    is a cross-section of the hydraulic pressure driven solute concentrating means and/or an dialysate concentrating means of an apparatus for generating dialysate for dialysis of Fig. 6 along lines XXIII-XXIII of Fig. 6.

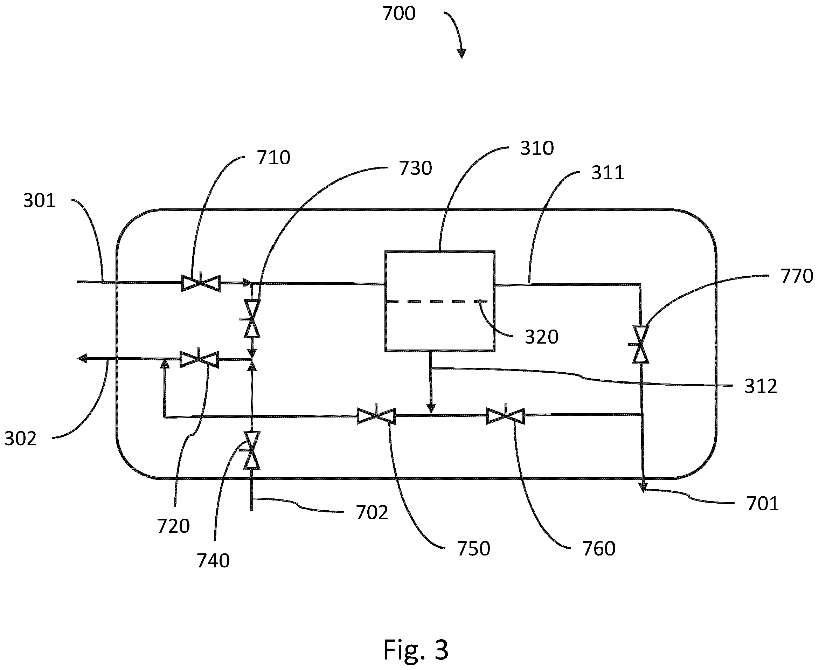

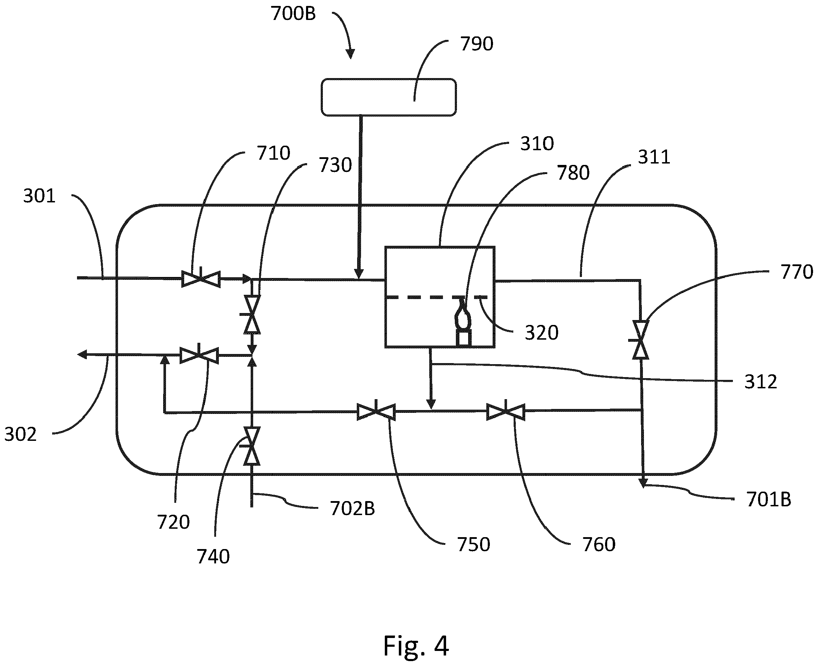

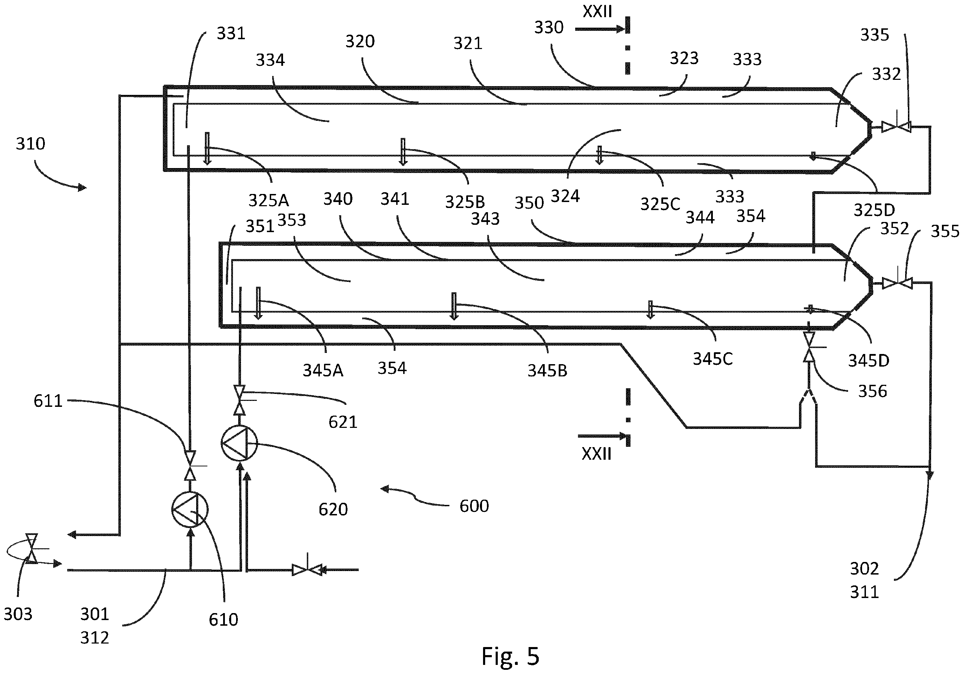

Figs. 1, 2, 16 and 21 are schematic diagrams of an apparatus 300 for generating dialysate for dialysis according to embodiments of the present invention. The apparatus 300 for generating dialysate for dialysis comprises solute concentrating means / dialysate concentrating means / a CMP based separation unit 310. Possible embodiments thereof are shown in Fig. 5 and 6. The apparatus 300 for generating dialysate for dialysis as shown in Fig. 1, 2 and 16 further comprises solute purifying means / dialysate purifying means / CMP based separation unit 700. Possible embodiments thereof are shown in Fig. 3 and 4.

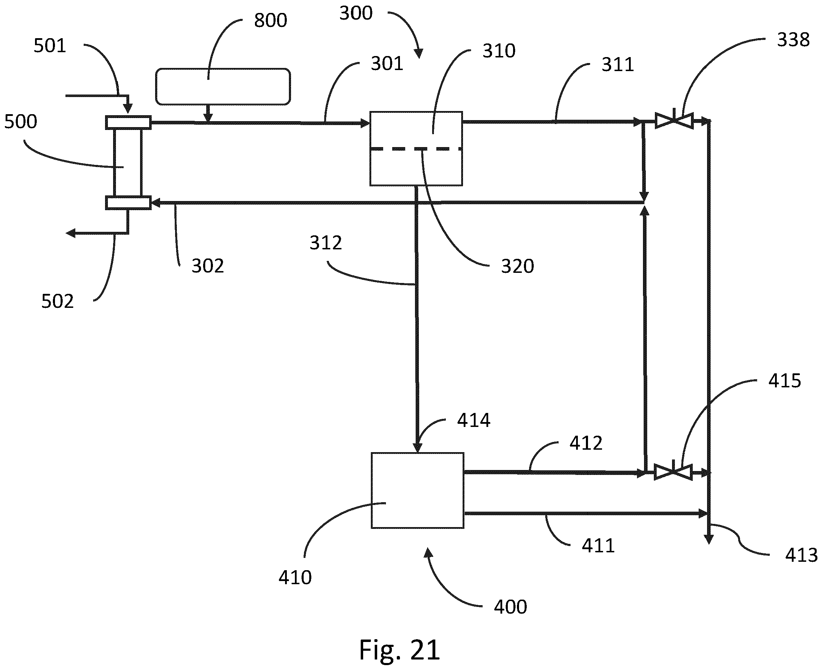

As shown in Fig. 21 the purifying means / dialysate purifying means / CMP based separation unit 700 is optional and has been omitted in the embodiment of Fig. 21. In embodiments without purifying means / dialysate purifying means / CMP based separation unit 700 the apparatus 300 for generating dialysate for dialysis comprises further valves 338, 415 for regulating the amount of fluid of return lines 311 to be fed back to dialysate return line 302 and to be discharged to final urine outlet line 413.

The apparatus 300 for generating dialysate for dialysis as shown in Fig. 1, 2, 16 and 21 further comprises water separating means 400. A possible embodiment thereof are shown in Fig. 7.

An overview of the component structure and principal flow connections is given prior to the more mathematical underlying functionality rationale of the components. It is helpful to gain a system overview first and therefore regard 310 of Fig. 1, Fig. 2 and Fig. 16 as a black box for now that will be explained later in detail with respect to Figs. 5 and 6.

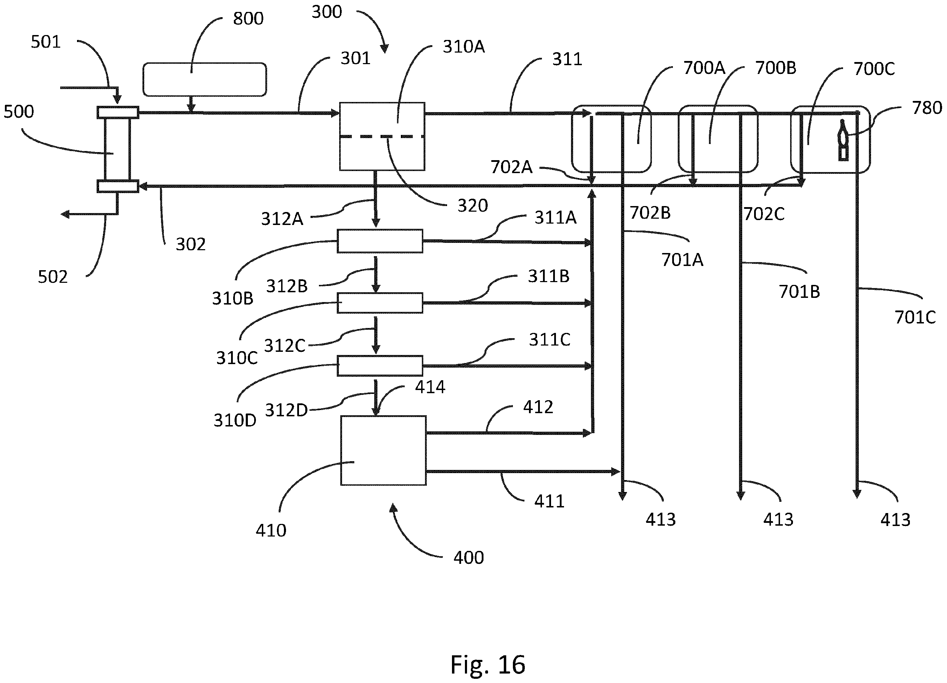

The invention in a first, typical but not exclusive embodiment can comprise a regular hemodialysis like setup (see Figs. 1, 2 and 16) with an arterial bloodline 501, a venous bloodline 502, and a primary fluid generation device 500 of any kind, e.g. a dialyzer that produces primary urine. The apparatus 300 comprises one (see Fig. 1) or more (see Figs. 2 and 16) solute concentrating means / dialysate concentrating means / CMP based separation units 310, 310A, 310B, 310B, 310C, 310D. If there are more than one solute concentrating means 310A, 310B, 310B, 310C, 310D, they are connected via flow lines 312A, 312B, 312C to form several concentration stages which can be equipped with identical or different types of semipermeable membranes 321, 341.

The water separating means 400 may comprise one or more cryo-based dialysate regeneration units according to EP 3 487 555 B1. The inlet 414 may be connected with the flow line 312 (Fig. 1), 312D (Fig. 2, Fig. 16) of solute concentrating means / dialysate concentrating means / CMP based separation unit 310, 310D.

The apparatus 300 for generating dialysate for dialysis 300 optionally may further comprise one (Fig. 1, Fig. 2) or more (Fig. 16) solute purifying means / dialysate purifying means / CMP based separation units 700, 700A, 700B, 700C. To provide these one or more further separation stages, solute purifying means / dialysate purifying means 700, 700A, 700B, 700C may be adapted and arranged similar or identical to solute concentrating means / dialysate concentrating means / CMP based separation units 310, 310A, 310B, 310B, 310C, 310D. The difference is that the diluate of separation stages, solute purifying means / dialysate purifying means / CMP based separation units 700, 700A, 700B, 700C is fed back to line 302, whereas the concentrate of solute concentrating means / dialysate concentrating means / CMP based separation units 310, 310A, 310B, 310B, 310C, 310D is fed back to line 302. The diluate of solute concentrating means / dialysate concentrating means / CMP based separation units 310, 310A, 310B, 310B, 310C, 310D is fed to water separating means 400. The separated water of water separating means 400 is also fed back to line 302. The number of solute purifying means / dialysate purifying means / CMP based separation units 700, 700A, 700B, 700C in the one or more purifying stages may differ as well as the membrane types may differ in all separation stages as even within a single separation unit.

The apparatus 300 comprises a dialysate inflow line 301 and a dialysate return line 302 sourced from return lines 311, 311A, 311B, 311C, 311D, 702, 702A, 702, 702C, 412 from the solute concentrating means / dialysate concentrating means / CMP based separation units 310, 310A, 310B, 310B, 310C, 310D, the solute purifying means / dialysate purifying means / CMP based separation units 700, 700A, 700B, 700C and the water separating means 400, respectively. The apparatus 300 also comprises drain lines 411, 701, 701A, 701B, 701C that combine to a final urine outlet line 413. Within all flow lines pumps may exist to establish the pressures needed to drive the fluids but are omitted in Figs. 1, 2, 3, 4 and 16 for sake of clarity.

Exemplary pump means 600 are shown in Figs. 5 and 6. There are pumps 610 and 620 having optional valves 611 and 612, respectively. Solute purifying means / dialysate purifying means / CMP based separation units 700, 700A, 700B, 700C may be equipped with similar or equivalent pump means.

Fig. 5 is an exemplary embodiment of solute concentrating means / dialysate concentrating means / CMP based separation units 310 provided in separate housings. There are two separate tubes 330, 350. Fluid is fed from line 301 and pressurized by pump means 600. Separate pumps 610, 620 or a common pump may be provided. Optionally valves 611 and 621 may be used for pressure regulation. The tubes 330, 350 comprise a first end 331, 351 and a second end 332, 352. In tube 330 there are semipermeable filter means 320 having a semipermeable membrane 321. At its first side 323 there is first flow path 333 corresponding to the ascending limb AL in a kidney. At its second side 324 there is a second flow path 334 corresponding to the descending limb DL in a kidney. Fluid (e.g. primary urine) is provided under pressure to the first end 331 of the tube 330 into the second flow path 334 such that the fluid flows from the first side 331 of the tube 330 to the second end 332 of the tube 330. The solvent is pressed through the semipermeable membrane 321. Arrows 325A, 325B, 325C, 325D show by its lengths the decreasing amount of solvent passing the semipermeable membrane 321 along its length from the second flow path 334 into the first flow path 333 such that concentrate is in the second flow path 334 and diluate is in the first flow path 333. At the first end 331 of the tube the diluate is taken from the first flow path 333 and provided to feed back 303. In tube 350 there are semipermeable filter means 340 having a semipermeable membrane 341. At its first side 343 there is first flow path 353 corresponding to the collective duct CD in a kidney. At its second side 344 there is second flow path 354 corresponding to the high osmolar interstitial environment near the papilla in a kidney. The solvent is pressed through the semipermeable membrane 341. Arrows 345A, 345B, 345C, 345D show by its lengths the decreasing amount of solvent passing the semipermeable membrane 341 along its length. At the second end 332, 352 of tubes 330, 350, there are respective valves 335, 355 for regulating the amount of concentrate to be taken out. The amount taken out of tube 330 is fed into tube 350. The amount taken out of tube 350 is fed back to feed back 303 or fed into line 302 and then venous bloodline 502 of the primary fluid generation device 500. Additionally any appropriate amount of concentrate may be taken out at any appropriate position from the second flow path 334 or 354, as exemplary shown in Fig. 5 with regulation valve 356. The setup according to fig. 5 allows to upconcentrate a solution A in tube 330 and to clear the same solution A or a different solution B in tube 350 like in the kidney where fluid composition at the Henle's loop bend and at the end of collection ducts may differ in composition but not in osmolarity. These two processes may be spatially separated. Fig. 22 is a section showing tube 330 with semipermeable filter means 320 separating the first flow path 333 corresponding to the ascending limb AL and the second flow path 334 corresponding to the decending limb DL. Tube 330 is spatially separated from tube 350 with semipermeable filter means 340 separating first flow path 353 corresponding to the collective duct CD and second flow path 354 corresponding to the high osmolar interstitial environment near the papilla in a kidney. This section is extending along the length of tubes 330 and 350. This basic structure is scalable and can deviate from a circular shape but is extending along the length of tubes 330 and 350.

Fig. 6 is another exemplary embodiment of solute concentrating means / dialysate concentrating means / CMP based separation units 310. Reference is made to the description of Fig. 5. Here the differences are described.

This embodiment has one tube 330. In tube 330, both semipermeable filter means 320, 340 are arranged. Tube 330 is enclosing or surrounding semipermeable filter means 340. Semipermeable filter means 340 enclose or surround semipermeable filter means 320. Preferably semipermeable filter means 320 and 340 are coaxially arranged. Semipermeable filter means 320 and 340 are arranged in a single housing, i.e. in tube 330. The first flow path 333 corresponding to the ascending limb AL in a kidney is inside semipermeable filter means 320. The second flow path 334 corresponding to the descending limb DL in a kidney is between semipermeable filter means 320 and semipermeable filter means 340 enclosing or surrounding semipermeable filter means 320. A third flow path (collective duct CD 336) corresponding to the collective duct CD in a kidney is between semipermeable filter means 340 and tube 330 enclosing or surrounding semipermeable filter means 340. At the second end 332 of tube 330 the fluid from the second flow path 334 is fed back into the first flow path 333 and a portion of the fluid in CD 336 is discarded via appropriate valve means (not shown in Fig. 6). The line to valve 357 can be fed with any fluid taken out of solute concentrating means / dialysate concentrating means / CMP based separation units 310 as described with respect to valve 356. Fig. 23 is a section showing tube 330 with semipermeable filter means 320 separating the first flow path 333 corresponding to the ascending limb AL and the second flow path 334 corresponding to the decending limb DL and semipermeable filter means 340 separating the second flow path 334 corresponding to the decending limb DL and the third flow path 336 corresponding to the collective duct CD in a kidney. This section is extending along the length of tube 330. This basic structure is scalable and can deviate from a circular shape but is extending along the length of tubes 330 and 350.

Semipermeable filter means 320 and 340 are arranged in the same tube, i.e. tube 330. Fig. 3 is an exemplary embodiment of solute purifying means / dialysate purifying means / CMP based separation unit 700. The working principle corresponds to the working principle of solute concentrating means / dialysate concentrating means / CMP based separation units 310. Here solutes are to be separated and/or to be removed such that the fluid is purified. By means of valves 702, 720, 730, 740, 750, 760, 770 it can be controlled to what extent the diluate is discarded or the concentrate is returned. Further valves may be provided as appropriate and in accordance with the knowledge of the skilled person.

Fig. 4 is an exemplary embodiment of solute purifying means / dialysate purifying means / CMP based separation unit 700B. Reference is made to Fig. 3. Here heating means 780 and/or pH modification means 790 are provided in addition such that the separation process may be promoted, for instance by moderately heating the ascending limb AL and/or the semipermeable filter means.

Fig. 7 is a schematic diagram of an exemplary cryopurifier 130 which may be used as water-urea separating means 400 and/or cryo-purifier / cryo-based dialysate regeneration unit 410 in accordance with the invention. The cryopurifier 130 comprises a crystallizer, i.e. a container for ice crystal growth 105. There is a cooling jacket 106 for cooling the content of container 105. The inlet 101 provides diluate from the solute concentrating means / dialysate concentrating means / CMP based separation units 310, 310D which is sprayed into container via dialysate inlet nozzle 102. The ice slurry 107 inside container 105 is stirred by stirrer 108 and discharged to feedback line 109 for optional seed preparation and slurry feed line 110 to the wash column 112. Feedback line 109 feeds a portion of the ice slurry 107 to crystallizer-heater-cooler unit 103 and then to seed crystal inlet nozzle 104. There are several valves 114 for flow control which are regulated according to the knowledge of the skilled person. The ice slurry 107 from container 105 is fed into wash column 112 via slurry inlet 111. A hydraulic piston 113 can be used to push slurry through the wash column 112, i.e. through liquid phase filter 115. At the bottom of wash column 112 there is outlet 116 for discharging concentrate. At the left side of the top of wash column there is a pure slurry pipe 122 through which solid and liquid is pumped in the direction of water outlet 125. A portion of the water is fed into water feedback loop 124 which is connected to the right hand side of the top of the washing column. Due to the pressure of pump 123 water is fed into wash column 112 and rinsing through the ice slurry in the wash column across wash front 118. Thereby concentrate is washed from the ice and concentrated at the bottom of wash column 112 such it can be discharged through concentrate outlet 116. Due to the wash front 118 there is essentially pure ice and pure liquid 119 at the top of wash column 112. At the top there is a rotating ice scraper 120 which scraped off the top layer of the wash front such that on top thereof pure slurry 126 is obtained which is fed into pure slurry line 122 via the suction of pump 123. A heater 121 can be used to generate water from the pure slurry.

The circuit can slowly be filled with ultrafiltrate extraction from the patient during a convenient period or initially by a separate priming amount means 800 provided at start of treatment.

## Functional explanation of the invention

It is not explained at this point how the setup described so far can perform truly the reclamation of water and also recover essential substances from the primary urine in 301 similar to mimic the native kidney nephron and return it in 302.

A basic idea of the functionality is that the setup addresses different molecular size fractions with different technical means. Uremic toxins (utox) size starts near water size with urea and ends somewhere at 60kD or above, just below albumin and globulin, transport proteins that must not leave the body in relevant quantities. Some of the utox are free floating in water (hydrophilic utox) at any time observed, others (lipophilic) are only a small percentage of all time off their carrier proteins by thermal collisions.

The standard method to separate all hydrophilic molecules of size > A > water size from its aqueous solution is to filter it using semipermeable membranes (SPM) with pores of size A to have the water pass and to establish an obstacle to the molecules with size > A. This separates all utox > A at the concentrate side. As water extraction through SPM by hydraulic pressure must overcome the more and more rising osmotic pressure that hinders the residual water from leaving, for purity high hydraulic pressure must be applied like in reverse osmosis (RO). This high hydraulic pressure tends to occlude the SPM pores with larger molecules, in particular if protein and their ions are present like in primary urine. To remedy this an additional flow tangential to the membrane is superimposed that leaves the filter system without having passed the membrane. This flow - called the retentate - is necessary to keep pores open and is discarded. It can be 50% of the input and therefore could be 90l/d when generating primary urine for humans, an amount the patients cannot refill by drinking. This is one but not the only reason why RO systems cannot replace this kidney function. And this is why solute concentrating means / dialysate concentrating means / CMP based separation units 310 does not work like regular RO systems.

Lipophilic molecules can only be filtered during the short percentage of time they are off their carrier proteins, thus prolonged filter time enhances their extraction by filter systems. It is clear if several filter stages are connected with subsequently rising pore size a filter system is able to separate molecular utox fractions of rising molecular weight by always letting the smallest utox fraction pass the membrane of a given stage.

Obviously molecules of water size (280pm) cannot be separated using membranes. Urea has about 300pm and cannot be filtered by pores effectively from water. Size is not a matching criterion for urea to be removed. Molecules significantly smaller than water could be removed by filters as well - but they do not occur within the utox. If size cannot be used as criterion, the ability to bend or stretch a molecule could be utilized. This is exactly what occurs when water ice forms: Any molecule that does not match the lattice conditions for best integration into the ice crystal in terms of bending, stretching and size has an energetic disadvantage versus water molecules that perfectly match the lattice structure. So has urea when ice forms, as well as all the other utox. They are repelled to the crystal surface by surface tension of the expanding crystal. To take advantage of this principle in particular if urea or a molecule of same size as the solution molecules is present the cryo-based regeneration unit 410 is integrated into the system - it can be omitted if all substances can be filtered, unlike urea from water. In combination with 410 all larger utox sizes as well as urea can be addressed. Unfortunately 410 extracts all substances, even those being essential. This is why it cannot be used alone and the total system must be combined with filter based separation stages with the flow volumes precisely balanced to match the medical prescription of dialysis. As described conventional RO filter technique cannot be combined with a cryo-purifier / cryo-based dialysate regeneration unit 410 for patient dialysis due to the high retentate flow. To implement this prescribed balance the functionality of a solute concentrating means / dialysate concentrating means / CMP based separation units / filter based separation unit 310 must be understood in detail - it works differently than a regular RO system.

Solute concentrating means / dialysate concentrating means / CMP based separation units 310 make use of the CMP principle like the kidney itself but the invention combines it in a convenient way with urea cryo separation of the EP 3 487 555 B1 family to mimic the nephron upconcentration without any biological or cellular activity. It is astonishing that even in the comprehensive 'Handbook of Membrane separations', PabbyAK, RizviSSH, SastreAM (Eds.), CRC, 2009, no explanation of the properties of a CMP separation unit and its differences to regular RO can be found. Countercurrent multiplication of single effects - CMP - is a generalized principle that can work not only with osmotic gradients but with many different type of gradients like gravity (in Zippe-centrifuges) or heat (in rete mirabili of some animals).

As mentioned the invention extensively uses the countercurrent multiplication principle within the solute concentrating means / dialysate concentrating means / CMP based separation units 310. This is the same principle the kidney benefits from, located at the Henle loop of the nephron, assembled to bundles within the kidney pyramids. This principle was first theoretically described correctly by Hargitay and Kuhn in Hargitay1951, a landmark paper written in German. (Das Multiplikationsprinzip als Grundlage der Harnkonzentrierung in der Niere, Hargitay B, Kuhn W, Zeitschrift fur Elektrochemie, 55(6),539pp, August 1951, engl. version: The multiplication principle as the basis for concentrating urine in the kidney, Hargitay B, Kuhn W, J Am Soc Nephrol 12, 1566pp,2001) and nowadays is fully accepted as correct theoretical derivation of the kidneys upconcentration differential equation.

This invention makes use of a technical insight Hargitay and Kuhn already had in Hargitay1951 when realizing that it does not really need ion pumps in cell walls to have the CMP work and found hydraulic pressure is completely sufficient. Accordingly all solute concentrating means / dialysate concentrating means / CMP based separation units 310 of the present invention may be driven by hydraulic pressure only, wherever needed a pump is inserted to generate it. Hargitay1951 derived the equations by only using hydraulic pressure, no sodium or potassium pumps in membranes have been assumed. On that assumption even a technical experiment that proved the principle without doubt is included in their 1951 paper. Using just hydraulic pressure is a central component of this invention that circumvents our inability to build membrane integrated, protein-based ion pumps technically. This alone allows to waive all cell biology in the invention. Hargitay1951 understood it leads to the same osmotic situation if an osmotic solvent is actively transferred across a semipermeable membrane from side A to B by cells using ion pumps and energy or if the water is transferred vice versa from B to A by hydraulic pressure only. Hargitay1951 demonstrated the technical ability of the CMP principle to concentrate sodium ions using cellophane membrane, sodium polyacrylate as solute and hydraulic pressure as driving force without doubt.

To understand the improvements possible when using CMP the differences to a present technical RO system have to be considered:

- The system has a hairpin bend configuration and returns all fluid concentrate at a hairpin bend so the fluid concentrate is not lost like with RO - although its amount at the hairpin bend is very small. This fluid is called concentrate and not retentate as is not detained from reaching the other membrane side. This is because

- The descending input limb (=proximal tubule) of the system is perfused so slowly by the input dialysate at convenient parameters like pressure, flow, cross section, length and membrane permeability that the water crossing the SPM forced by hydraulic pressure at a location x and thus subsequently establishing a counteracting osmotic pressure to outweigh the forcing hydraulic pressure at any x along the hairpin configuration will always have sufficient time to balance an essential part of the hydraulic pressure applied before propagating further along x, except a small residuum. The flow is chosen such that there will always remain a little osmotic and hydraulic pressure difference, so that a little further water will pass the membrane at any x. This means at any location only a small osmotic and hydraulic pressure difference is physically effective and the water in the descending limb (DL) at the neighboring location x-dx is only of slightly lower concentration than at x and then propagates from x to x+dx while slightly increasing concentration on one DL side of the SPM, to x-dx slightly decreasing concentration on the ascending limb (AL) side. As water is passing the membrane the flowrate decreases when approaching the hairpin tip and if at all only a small fluid amount will really return via the hairpin bend, most of the water will return long before reaching it at some location x along the path. But all solutes bigger than the pores cannot pass the semipermeable membrane at any x and completely reach the hairpin tip and return within the AL afterwards. So a very high concentration is established at the hairpin tip without high concentration differences across the SPM at any x. This is why the cells in Henle's loop although residing at locations with very high concentrations near the papilla do never see too strong osmotic gradients to destruct them. With the length of the system chosen conveniently there will be very high concentrations achievable (40fold possible) at the hairpin bend but only very low flows. That is exactly what happens in Henle's loop.

- After passing the hairpin tip and return to the distal end of the AL the fluid will reach nearly the same concentration as at the inlet, because the water that passed the SMP now dilutes the high concentrate that came from the hairpin tip up the AL with exactly the amount of water that has been extracted during its way down the DL. So the AL outlet nearly has unchanged concentration compared to inlet of DL using a CMP configuration as described. The outlet of a RO system however is changed. The system as described generates high concentrate at the hairpin but also consumes it again.

- As only small hydraulic pressure differences apply to any membrane segment because osmotic pressure nearly outweighs it the small flow at any x is sufficient to prevent membrane fouling. The flux per SMP area unit is much smaller. Large molecules and proteins are not deeply pressed into the membrane by extreme hydraulic pressure like in technical RO systems. In a hairpin configuration fouling is not an issue as there is very small hydraulic pressure difference across the membrane, but the specific water flux per SPM area is much less than in RO.

**The effect of fluid removal at the hairpin**

At this point - when filling in waste dialysate to the DL and having return the same amount of same concentration from the AL nothing seems won except that a region of very high concentration near the hairpin tip has been created. But this region of high osmolarity actually is a great advantage. If we imagine to bundle many such systems a complete region of high osmolarity can be established like close to the papillae renales in the kidney. If we further take into account that osmolarity is a colligative property that just looks at molecule counts and not at molecular types, there is a further option: We can attach a further (collective) duct CD alongside the already considered DL and AL with a further SPM2 - even of different pore type - in close contact to the DL/AL system with not a hairpin bend but an open orifice at the distal CD end that allows some fluid to leave the system at the location of high concentration. The input fluid within this CD (it's the analogue to the collective duct in the kidney) can originate from the distal AL outlet after having passed the first two limbs completely or from any different pool elsewhere. Water is transferred across SPM2 only according to the conditions established at both sides by the respective osmolarities. So on the first side this is essentially the CD inlet osmolarity and its development along the flow path, on the other side it is the action of the DL/AL system as described above. This means the new limb CD is not more than an osmotic load to the DL/AL system, possibly supplied with different types of molecules. As long as the DL/AL system is able to keep the high concentration a specific amount of water is extracted from the CD via SPM2 to equilibrate osmolarity. At the outlet of the CD orifice a high concentrate with possibly different composition as at the hairpin tip but nearly the same osmolarity will appear. In the kidney exactly this CD outlet is called secondary urine that leaves via the ureter. With the CD added the solute concentrating means / dialysate concentrating means / CMP based separation units 310 have at least 3 connections as depicted in Fig. 1, Fig. 2, Fig. 5, Fig. 6 and Fig. 16: An inlet 301 to DL, an outlet 312 from AL and the high concentrate outlet from CD 311. SPM2 is not depicted as its distance to SP1 is on a micron scale. The fourth connection to CD inlet is not depicted. In a practical setup in most technical cases but not mandatory this inlet will be the same fluid as DL inlet or AL outlet, thus both coming from the 301 inlet which can be a double inlet to both.

Such solute concentrating means / dialysate concentrating means / CMP based separation units 310 as explained can be used to extract a small, still unknown amount of fluid with high solute concentration - up to 40fold of CD inlet- for solutes separable by membrane pores to line 311. It can also be used to extract the remaining water of higher purity at the AL from line 312, as the solutes leaving at the 311 output are missing there. Idealistically the water at 312 will still contain the full content of those molecules coming in at 301 that are smaller than the pores. These molecules could not be separated from the water and therefore behave like the water (or solvent) itself. In conclusion for these molecules that can fully pass the membrane the CMP does not work at all, for those passing partially (0< separation <1) the CMP works to a reduced extent only. In the AL outlet fluid at 312 the concentration ratio between those substances that could fully or partially pass the membrane like urea on one hand and those like the bigger utox that cannot pass the membrane at all on the other is shifted in comparison to the inlet ratio at 301. This is an essential property of the setup we will make extensive use of: As not all but a small amount of highly concentrated fluid was extracted at 311 (the CD orifice) this is missing in 312 and therefore the concentration ratio of the permeable and nonpermeable solutes is larger at 312. Vice versa at the 311 outlet the same ratio is smaller as the permeable solutes are missing to a certain extent. In general this means at the AL outlet 312 the smaller solutes are elevated, at the CD outlet 311 the larger solutes are elevated. Such a CMP separation unit can be used to shift the concentration ratio between small and large solutes. This is helpful when it comes to produce mixtures of shifted concentration ratios compared to inlet and an essential approach to control the composition of the outlet secondary urine.

It is clear that one or more separation stages with different membrane pore size can be switched in sequence to enhance or even separate a desired size fraction of molecules if all of them are bigger than the solvent molecules. In particular electrolytes and bicarbonate could be separated from bigger utox this way using a first stage 700 (fig.3). This is recommended if their balance must be controlled individually and independent of other utox. Depending on salt and electrolyte intake of the patient the electrolyte removal could be prescribed to be so low that the accompanied bigger utox are not removed sufficiently if they are kept in same concentration ratio as they come with the primary urine. In this case a first separation stage 700 would be used with a pore size designed to separate small electrolytes and bicarbonate from bigger utox. In this case also the electrolytes and bicarbonate would pass the membrane like water (it was not a SPM to them), are elevated in the AL outlet and return in a desired fraction at line 702 to the body in 302. The bigger utox however are removed via 701 to the ureter.

**Combining CMP and cryo-purification**

We did not yet address substances of same molecular size as the solute molecules. In particular none of the CMP separation units described so far are feasible to separate urea. At this point we note that only water separating means 400 in accordance with the present invention and especially the cryo-purifier / cryo-based dialysate regeneration unit 410 are/is able to extract urea from water - in general and if freezing works - solutes of same size as the solution molecules, but it moreover extracts all other types of molecules additionally. It will not totally extract but reduce all existing solutes by the same percentage which is in the range of 90-95%. It produces 95% of the water with 5% of solvents at line 412 and 5% of the water with 95% of the solutes at line 411. The ratio of the solutes is not changed by 400. This helps to change the relative composition. Compared to CMP this is a different operational principle with similar but wider effect as it also includes those solutes of solvent size. According to one aspect of the present invention it is a central concept to realize electrolyte and bicarbonate recovery by combining cryo-purification for substances that cannot or only weakly be addressed by filters with filter based CMP separation units that work like described above. The functionality of cryo-purifier / cryo-based dialysate regeneration unit 410 is described in EP 3 487 555 B1, the complete disclosure thereof is incorporated herein by reference. A cryoregeneration property is that any non-water or in general any non-solvent molecule is extracted at similar efficacy but not for 100% - outstanding purity cannot be reached with cryo-purification like with filters.

**Balancing the purification stages in a linear combination**

As a cryo-purification unit links the extraction factor of all solutes removed to the more or less same value, the case may occur that the solute component with a size of the solvent molecule, for which in particular the cryo-purification unit is implemented, needs a different removal factor than other solutes. In medical dialysis the human urea plasma concentration is about 7mmol/l (often more). Approximately 25g/d (412mmol/d) urea have to be excreted within 1500ml/d of urine. Thus the natural urine will typically have 275mmol/l. Compared to plasma this is a rise of roughly factor 40. This is different for NaCl: 150mmol/l is a typical concentration in plasma water. It depends on the dietary intake, physical activity (sweating) and of the medical prescription the patient has to obey. Typical the WHO recommends an intake of not more 5g/d (86mmol/d). Patients do not always obey their prescription and it is realistic that a technical system according to the invention will have to remove up to 500mmol/day of NaCl (29g), solved in 1500ml of secondary urine, this is 330mmol/l, which is a factor of roughly 2 only, but not 40. During strong antidiuresis a concentration up to 700mmol/l (for Na+ alone) may occur, thus a Na+ upconcentration factor of 700/150=4.7 was needed and must be delivered by the system. This is a condition that can easily be avoided by behavior and diet but marks the technical range to be covered by the invention. Urea must be cleared 40/4.7=8.5 times better than NaCl by a setup according to the invention in dialysis.

This consideration demonstrates that a cryo-purification unit alone - adjusted to remove sufficiently urea as prescribed - would also remove 40 x 150mmol/l *1.51/d=9000mmol/d = 525g/d NaCl, assumed that every Na+ takes an Cl- with it and thus cannot solve the problem to recover electrolytes and other valuable solutes as intended by the invention. An adult man with 70l total body water would lose more than 80% of his total body NaCl during one day. This is why in the example the water must be reduced in its NaCl content down to 4.7/40=0.117 (11.7%) vs. plasma before entering the cryo-purifier / cryo-based dialysate regeneration unit 410.

This can be performed by the solute concentrating means / dialysate concentrating means / CMP based separation units 310. The effective molecular size of Na+ is larger than its ionic size due to hydration. So some of the uncharged small uremic toxins of similar molecular weight like Na+ (23) are able to pass the membrane where Na+ does not. They will essentially appear in the urea fraction therefore and also experience upconcentrations in the same range of 40 by the invention. This is sufficient for e.g. acrolein (56), p-cresylsulfate (31), phenol (94), thiocyanate (58) and many other small uremic toxins (Normal and pathologic concentrations of uremic toxins, DuratonF, CohenG et al, JAmSocNephrol23:1258, 2012). As some of the larger proteins need higher upconcentration than sodium (DuratonF et al) it can be helpful to have another unit 700 to separate the larger fraction from the smaller utox and have it more upconcentrated before discarded to drain. HCO3-(bicarbonate anion, 61) as part of the essential pH buffering system is the only solute in this mass range that must not be removed to unacceptable extent. This slightly adverse effect of the invention can be compensated still by oral intake, no admixture to the returning dialysate is needed: HCO3- has a slightly larger stokes radius (relevant for pore passage) than Na+ and therefore will be permeated less than Na+ at same hydration, further a slight Donnan effect might repel anions from the membrane. Na+ removal is a fixed value prescribed by a doctor. In a typical situation a patients 1.51 urine concentration is 220mmol/l NaCl to remove 20g of it. The plasma concentration is 150mmol, thus the overall NaCl upconcentration of the setup is factor 1.5. Bicarbonate plasma concentration, typically 25mmol/l, concentrated by same factor of 1.5, will remove 25mmol/l * *1.5* 1.51/d =56mmol/d as worst case if it could pass like Na+, which is not the case. This still can and must be balanced by oral intake of not more than 5g NaHC03/d when using this system for dialysis if a membrane type is used in 700 that cannot distinguish between Na+ and HC03- at all. If the membrane can distinguish to a certain extent between their stokes radii the oral substitution will be proportionally less. This is still practical and therefore the invention can cover the full range of utox and withhold all relevant substances sufficiently. As there are different flow paths combined that are originating from different purification methods and concentrate levels (701, 311, 411, 412) it will be of relevance to find the correct fluid amounts for admixture from any flow path. The prescription for sodium and urea as the guiding criterion will be matched of a linear combination from different flows. This linear combination will be mathematically established and controlled by valves in any flow line (valves not indicated for simplicity). After having given an overview of the total system setup it is necessary to have a closer look to the CMP separation units that have been regarded as black boxes for now to understand the fluid management behind it.

**A deeper explanation of the setup**

Membrane based separation units working with CMP and cryo-purification unit have different properties. This must be elucidated in detail to follow the rationale of fig 3. These properties may differ in efficacy and grade of separation dependent of their constructional parameters (pressure, flow, interaction surface, separation, specific transferability, length and size, configuration).

Moreover Hargitay1951's theoretical considerations are based on only 1 solute (sodium). They do not reflect simultaneous filtration of two or more different osmotically active substances. Urea that can only be separated with separation (S) of 0.2 on technical membranes will behave different from sodium. S=0.2 means if 100 molecules 'ask' the SPM for transfer permission only 20 are rejected, 80 pass it. If in the same setup a solute with S=1 (e.g. sodium) is separated completely this results in different operational parameters (size, pressure, duration, throughput) for the same concentration factor. This can be one of the deeper reasons why a kidneys nephrons are of so different length in the same kidney. It is important to enhance Hargitay1951's considerations on a CMP system when acting with two different solutes, in particular urea with S=0.2 and sodium with S=1. As both contribute to osmolarity and therefore influence each other a further calculation that helps to understand how this impacts build sizes, pressures and process throughput will be necessary. The mathematical approach, starting at Hargitay1951's paper, is needed to understand functionality of the invention.

A most important difference of Hargitay1951's analysis and the kidneys working principle is that Hargitay1951 used hydraulic pressure that works against osmotic pressure (like in RO devices) to separate the water. He used the equivalence that it does not matter to a systems osmotic situation if water is pressed in one direction by hydraulics (like in his calculation) or if sodium is pressed in opposite direction by ion pumps (like in the kidney). The osmotic result finally is the same and the calculation as well as the technical solution can be accomplished using hydraulic pressure instead of ionic pumps without any difference in the result. As mankind not yet has invented reliable technical ion pumps or urea transport proteins in thin semipermeable membranes -and therefore cannot build a true technical kidney - this invention relies on the opposite principle by using hydraulic pressure instead of ion pumps. The result is the same but it circumvents involving living cells like some of the prior art solutions do. Hydraulic pressure can easily be generated technically by pumps. It enforces a certain mechanical stability however (which the kidney tubules do not have, so they would burst under hydraulic pressure) but much less stability than RO membranes need.

Hargitay1951 formulated the basic differential equation of upconcentration at a countercurrent multiplication device like the nephron is. This equation rules the nephrons behavior or any other S-formed device with semipermeable membranes within its limbs:

$$\left[\frac{dv_1}{dx}\right] = -\frac{\gamma p}{au_{10}}\left[v_1^2 + v_1^3 \frac{\xi(v_l - v_1)}{P(\xi v_1 - 1)}\right]$$

3.8 of Hargitay1951

The variables are defined in the following image. It is a modified image originating from Hargitay1951's image 6b and describes the internal structure of the solute concentrating means / dialysate concentrating means / CMP based separation units 310 in Figs. 5 and 6. The flow enters DL with pressure p, the pressure p drops completely at the hairpin turn so that throughout the full length the pressure p is applied to the membrane - but is reduced by the local osmotic pressure difference. How this osmotic difference and therefore the flow develops is subject of Hargitay1951's calculation. After turning at the hairpin bend the flow leaves via AL -which corresponds to line 312- and may be returned into CD, where it finally leaves the system at the right end, which corresponds to line 311.

Hargitay1951's equation 3.8 is not easy to understand. It is possible to roughly formulate some properties of Hargitay1951's equation 3.8 in words:

The change per length of the concentration factor $\nu_1$ in the DL along the length L at a location 1 is proportional to a sum of the square and the $3^{rd}$ power of $\nu_1$ whereas the third power is multiplied by an expression that only depends on flow parameters relevant to the kidney. Hargitay1951 has chosen these kidney relevant parameters appropriately:

$\varsigma$ is the fluid fraction entering CD of the total fluid entering CD and DL.

P is the ratio p_hydraulic/p_osmotic at the entrance to DL, CD at x=0.

$\xi$ is the fluid fraction leaving CD of the total fluid entering CD and DL. This corresponds to the kidneys secondary urine fraction which is roughly 0.5%.

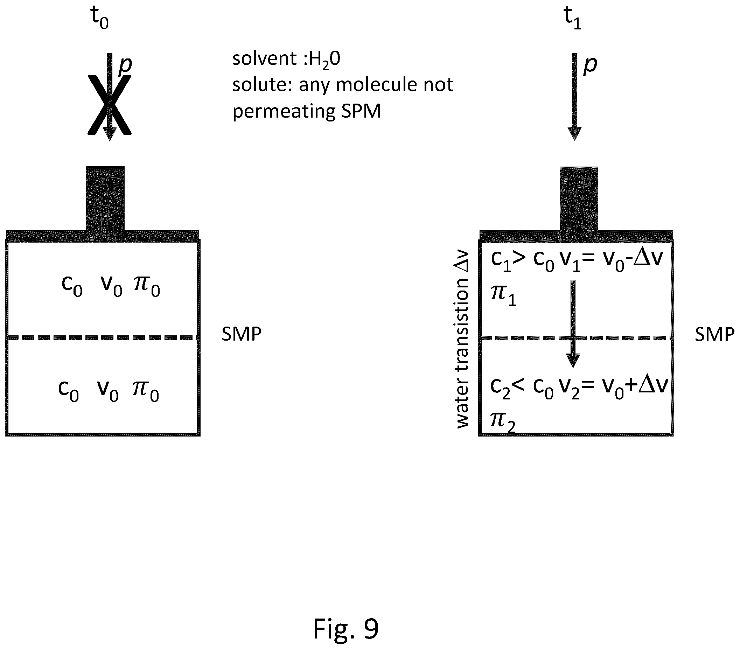

To intuitively understand the infinitesimal limit transition for reading the equation 3.8 it is helpful to make a thought experiment with finite steps of differences >0 that later are approaching zero - as usual if a differential expression is formed. Assuming a finite segment of the setup depicted in Fig. 8 is magnified and initially has concentration c0, volume v0 and osmotic pressure n0. If a pressure p is applied across the SPM (Fig. 9) we observe a water transition caused by p. This was called the single effect by Hargitay1951.

The water transition decreases v and in consequence increases c in the upper compartment (and vice versa in the lower) and therefore the osmotic pressure $\pi$ counteracting p rises until p is equilibrated as $\pi$ acts in opposite direction to p. This step in the kidney is done by ion pumps transferring sodium - technically water is transferred. This makes the principle of the kidney technically feasible at all. At equilibration the water flux across the membrane stops. Both differ the same spreading from the initial value.

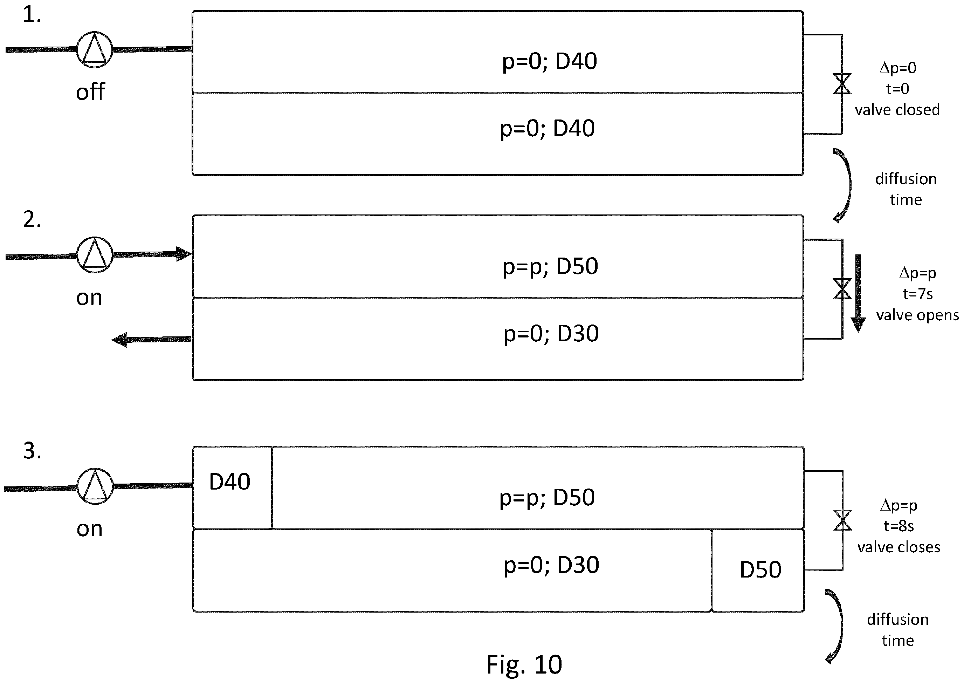

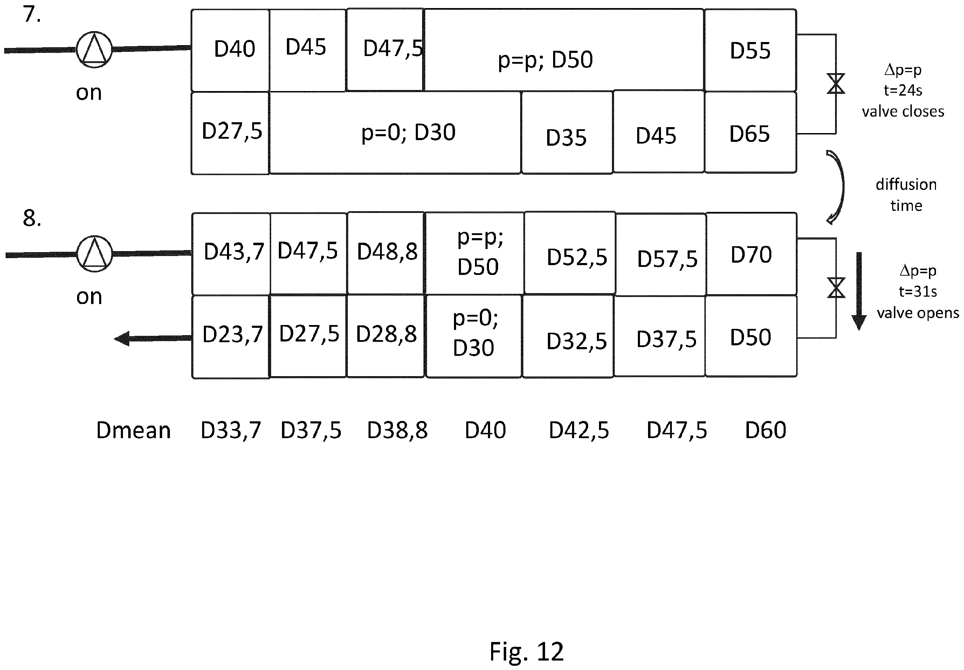

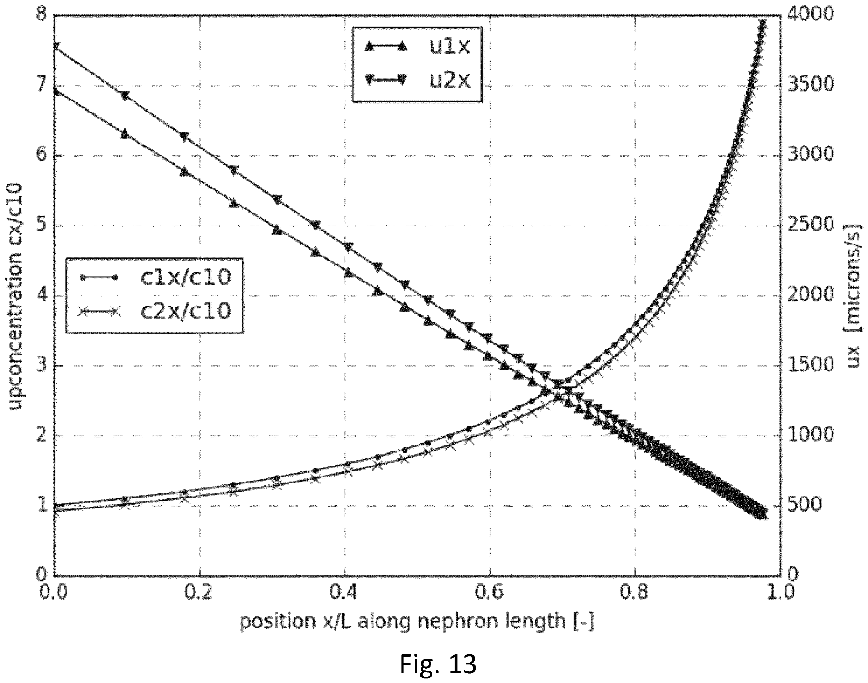

In the next step the thought experiment is enhanced by putting several of the units of fig 13 aside each other to form a pipe without any wall separating them at the left and right (Figs. 12 to 12). We start in a situation the full setup as depicted in Fig. 10 is filled with solution containing molecules that cannot permeate. After filling at any point the same concentration exists. A fictive concentration is assigned, indicated by grey shade density D40 initially. A pump presses water with concentration D40 into the setup whenever a valve at the hairpin bend is open. Hydraulic pressure drop at the hairpin bend is - idealistically - complete, in the AL (the lower) p=0 is measured. We assume - just arbitrarily - within 7s SPM diffusion has completed but geometry is designed such that diffusion is too slow to reach the neighboring cell significantly, as convective flow dominates transport along x.

The element of Fig. 8 is packed 7 times aside each other. As we switch on the pump (Fig. 10, steps 1. and 2.) with the setup filled without pressure already prior to start of the thought experiment the full length will work as a row of the unit in Fig. 8. The upper DL will increase concentration for the same amount as the AL will decrease concentration after 7s diffusion time. If the valve now opens for 1s the volume elements will proceed for 1 position without relevant diffusion into previous or subsequent elements - this means the 1s is regarded to be very small compared to 7s, which is idealistic. The element directly before the hairpin turn will wander down into the AL and is in opposition to its previous successor in the DL.

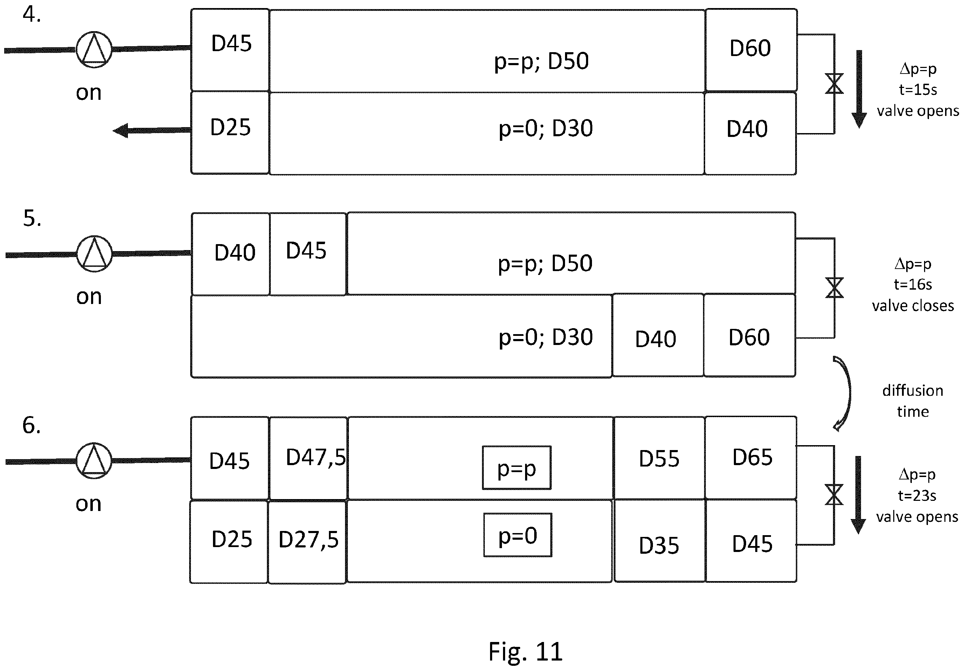

If this sequence - diffusion time elapse and proceed one $\Delta$ x - is repeated several times (Figs. 10, 11, 12, steps 1. - 8.) it becomes apparent that at the right end a significant upconcentration occurs - just by hopping one position, calculate the mean and a symmetric concentration spreading difference and then do the hop again. This can reach 40fold upconcentration easily and depends on the geometry, flow, membrane and length in particular. Stripping down the process this way by just performing the limit transition $\Delta$ x ->0 for the integration makes the 'wonder' of the kidney intuitively understandable and using hydraulics instead of ion pumps makes it technically feasible. Once a region with high concentration is established this way it can be imagined that a third CD pipe attached to the region will extract water by forward osmosis from this additional pipe, independent of what molecules are used as osmotic agent within CD. So the solutions used in DL and CD may differ or not. This is what the kidney uses when extracting water from the collective duct in its inner regions that are upconcentrated using this mechanism.

**Numerical simulation of Equation 3.8**

Hargitay1951 has published the complete mathematics as well as an experimental proof and it is beyond this scope to delineate it here as it is published and accepted, proven knowledge of the skilled person. But it will be applied to urea in this disclosure which application is not known so far. As differential equation 3.8 cannot be solved analytically a larger numerical calculation has been conducted that solved differential equation 3.8 in the presence of both sodium and urea to find out what properties such a system has and if it could be built in combination with a cryopurifier. The essential results from this calculation are presented here to demonstrate that the size of the setup can be handled in dialysis or other environments even for wearable dialysis.

The numerical model in principle was designed according to the mechanism depicted in Figs. 10-12. The third pipe, CD, was internalized to DL, this means it was calculated as a part of DL containing the same solution to avoid another membrane that would extend calculations without additional information. To keep calculation short only 10 or for comparison purposes 20 steps in x have been implemented. A comparison to the analytical solution of 3.8 using SageMath version 7.4 (www.sagemath.org) was made based on sodium alone to estimate the effect of simplification in discretization versus the integration limit transition. An analysis was made to see when the system will become stable. It was found that usually the system was stable after 100 calculation steps, so in all numerical runs 400 calculation steps like in Figs. 10 to 12 were used.

The length L of the setup was 20cm and the membrane had assigned a permeability typical to technical RO membranes. The numerical analysis was performed using excel® 2016. Within numerical analysis for each of the AL, DL side all particle numbers, the concentrations [NaCl], [urea], the total and partial osmolarities, the total and partial osmotic pressure $\pi$, the volume according to the single cell effect, further all of them at the initial situation after proceeding one step (valve open) and after elapse of diffusion time (valve closed) have been calculated. Further the transfer quantities that rule the transition from initial to final situation of a discrete calculation step were determined. These were effective pressure difference $\Delta p$, initial flow, transfer volume of water and also transferred urea molecules depending on a separation S (S=0.2 was used for urea, S=1.0 for sodium), mixing concentration of all osmotically active particles and the urea based fraction of it as well as the diffusion time. For diffusion time calculation the initial_flow/2 and the transferred volume was used. When urea is present diffusion time differs with x. The two longest times near the input/output have been discarded to avoid ineffective waiting. Diffusion times have been used to find out which flow was possible with a given setup and how many parallel systems are needed for a given total clearance. Diffusion times have been added for all calculation steps - even if stability was achieved prior to the fixed 400 steps. This means that the timing result is too long in the sense of a worst case and the separation process will be quicker as calculated.

The mass balance of all steps was calculated and used as quality criterion against calculation errors - as no particle may vanish to or appear out of nowhere. Calculations were accepted only if mass balance was maintained within the excel rounding error. Some aspects of the numerical model are compared in the subsequent section as it will be used finally for the rationale of the setup depicted in any of Figs. 1, 2, and 16. First a comparison with respect to sodium is done so that the numerical model finally can be enhanced to urea also. Hargitay1951's model, the analytical and numerical model were compared without urea because the first two models did not include urea. The analytical model revealed the following curves for concentration (see Fig. 13; c1x/c10 and c2x/c10) and flow velocity (see Fig. 13; u1x and u2x) using the parameters of table 1.

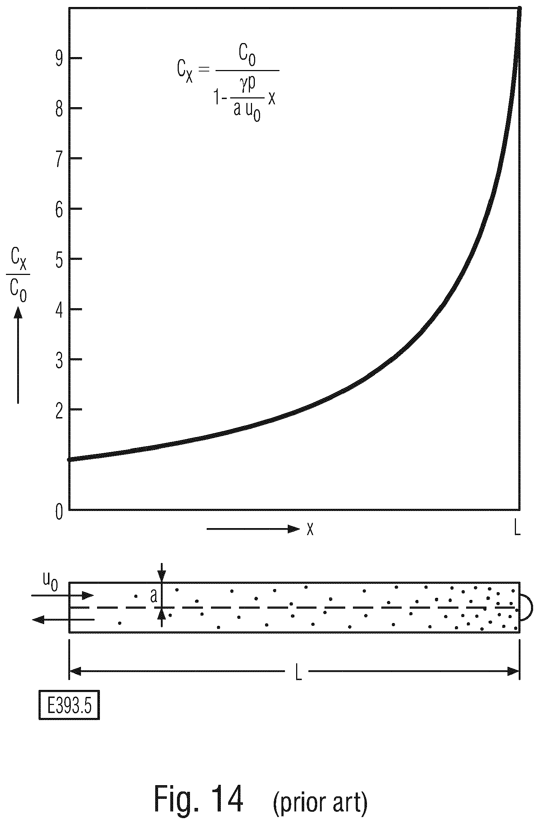

The curves for concentration are similar to the result of figure 5 of Hargitay1951, which is shown in Fig. 14 of this disclosure.

The same situation was analyzed by the numerical model (Fig. 13). Depicted are the values at the end of diffusion time:
The numerical model shows similar but not identical concentration curve. The endpoints coincide, the area in between does not. The deviation can be justified by a specific imputed difference caused by the properties of the numerics chosen:
The analytical model calculates using variable flow speed along the fiber (see ulx, u2x in Fig. 13)- as it truly is. Variable flow speed would mean to vary $\Delta x$ depending on flow speed after each calculation step. Variable $\Delta x$ means variable cell position. But in excel® the position of a cell is fixed so variable flow speed cannot directly be implemented if technically a cell in excel® denotes a specific location and there are not thousands among them to choose from. Therefore the $\Delta x$ was fixed in the numerical model. If it was variable this would result in an exponential curve, the $\Delta x$ at small x would be longer like with the analytical model (see Fig. 13) and became smaller towards L. This would make the simple excel® model much more complicated and was avoided as only the endpoints are of interest to proof a technical approach for a desired upconcentration. With this in mind it is acceptable to suppose the numeric model yields same result for calculations based purely on sodium concentration at the endpoints. This was further proofed making a more dialysis related comparison of the analytical and numerical model. The purpose was to find out if the deviations caused by discretization and constant $\Delta x$ are acceptable in a dialysis situation, e.g. on a wearable device with low clearance (table 1).

It can be seen (table 1) there are some deviations between analytic and numeric calculation with a given example but many of them do coincide to an unexpected extend and none differs more than a factor of 3, even not the permeated surface that strongly depends on $\Delta x$. Without urea the simple, roughly discretized numerical model seems to match the analytic calculation sufficiently so that further considerations were performed by using it. Moreover both numerical and analytical calculations are expected to deviate from an experiment in any case. A certain difference is accepted as numerical models can never represent reality completely accurately. The numerical model now was enhanced to also include urea.

table 1

| Calculation model | **analytical** | **numerical** |
|---|---|---|
| Cross section: | 1,1E-8 m$^2$ | 1,1E-8 m$^2$ |

(continued)

| Calculation model | analytical | numerical |
|---|---|---|
| Clearance desired | 20,8ml/min | 21ml/min |
| Fiber length L | 20cm | 20cm |
| Membrane-permeability | 7,2 L/m2/h/bar | 7,2 L/m2/h/bar |
| Dimensions: width × height | 110μm(AL,DL+CD) × 100μm | 110μm(AL, DL+CD) × 100μm |
| Pressure | 8800hPa | 8809hPa |
| Concentration [NaCl] | 155mmol/l | 154,23mmol/l |
| Upconcentration factor for NaCl at inlet(0) and tip (L) | 8 | 9,32 |
| # modules needed for clearance | 10000 | 13280 |
| Total length of fiber | 2000m | 2650m |
| Permeated surface | 0,8m$^2$ | 0,266m$^2$ |
| flow velocity | 3470μm/s | 2395μm/s |
| Urine output | 375ml/d | 294 ml/d |
| Travel time for L | 58s | 83s |
| Time for stability after startup | appr. 500s | appr. 1200s |
| NaCl in urine | 58,1 mmol | 45,4 mmol |

**Numerical calculation including urea results in a possible setup for dialysis**

table 2

| 360 | ml/min | Dialysate input flow (not clearance!) |
|---|---|---|
| 0 | | S, urea separation of membrane (treats urea like water) |
| 1 | | S, NaCl separation of membrane (separates NaCl compl.) |
| 2,00E-12 | m3/s*1/m2* 1/Pa | γ membrane permeability |
| 7,2 | l/m2/h/bar | γ membrane permeability |
| 827629 | Pa | hydraulic pressure in first level |
| 0,04 | mm | a, width of a rectangular shape of ascd./desc. limb AL, DL |
| 0,04 | mm | b, width of collective duct CD |
| 1 | mm | h, height of AL, DL, CD |
| 1 | mm | dx, length discretization step |
| 10 | mm | L, length of each ‚technical nephron' |
| 150 | mmol/l | [NaCl] in blood plasma |
| 7 | mmol/l | [urea] in blood plasma |
| 500 | mmol /d | NaCl removal (29,2g/d) |
| 412 | mmol/d | urea removal (25g/d) |
| 1120 | ml/d | fluid removal |
| 30 | - | upconcentration factor of cryo purification |

The numerical calculation described so far was enhanced for urea, using the principles as described above. Purpose of the calculation was to find out if the combination of CMP according to the model described and cryo-purification according to EP 3 487 555 B1 would allow to build a technical system that can provide dialysis water without dehydration and concentrate / bic loss. It should work even with a 100kg male patient at too high salt intake and low fluid overload. It should recover electrolytes and bicarbonate sufficiently so that only an acceptable oral administration of bicarbonate is necessary.

To anticipate, the setup of Fig. 1, 2 or 16 is the result of this numerical calculation.

The assumptions as outlined in table 2 were made for the calculation.

The result of the calculation including the mass and fluid transfers for NaCl and urea as well as their concentrations is in accordance with table 3, wherein reference is made to the setup of Fig. 16 when using available membranes and the cryo-purifier / cryo-based dialysate regeneration unit 410.

table 3

| reference numeral | | NaCl | | Urea | |
|---|---|---|---|---|---|
| | l/d | mmol/l | mmol/d | mmol/l | mmol/d |
| 301 | 44,38 | 154,23 | 6844,7 | 14 | 621,3 |
| 302 | 43,26 | 146,67 | 6344,9 | 4,84 | 209,4 |
| 311A | 4,33 | 790,5 | 3422,9 | 14 | 60,6 |
| 311B | 3,79 | 452,13 | 1713,6 | 14 | 53,1 |
| 311C | 3,24 | 263,9 | 855,0 | 14 | 45,4 |
| 311D | 2,69 | 159,26 | 428,4 | 14 | 37,7 |
| 312A | 40,05 | 85,45 | 3422,3 | 14 | 560,7 |
| 312B | 36,27 | 47,19 | 1711,6 | 14 | 507,8 |
| 312C | 33,02 | 25,91 | 855,5 | 14 | 462,3 |
| 312D | 30,34 | 14,1 | 427,8 | 14 | 424,8 |
| 411 | 1,01 | 408,97 | 413,1 | 406 | 410,1 |
| 412 | 29,33 | 0,49 | 14,4 | 0,48 | 14,1 |
| 701 | 0,11 | 790,5 | 87,0 | 14 | 1,5 |
| 702 | 4,22 | 790,5 | 3335,9 | 14 | 59,1 |
| 413 | 1,12 | 446,02 | 499,5 | 367,93 | 412,1 |

Solute concentrating means / dialysate concentrating means / CMP based separation units 310B, 310C and 310D an water separating means 400 return 39,04l/d (77,08mmol/l NaCl and 3,85mmol/l urea) to line 302 (in addition to the return from line 702). Solute purifying means / dialysate purifying means / CMP based separation unit 700 discharges 86,95mmol NaCl and 1,54mmol urea to urine as it is designed for bigger molecules and therefore urea and sodium concentrations are not modified by unit 700.

In total there is a removal of 500mmol (29,2g) NaCl, 412mmol (25g) urea in 4 stages of solute concentrating means / dialysate concentrating means / CMP based separation units 310. The excretion is 1,12l/d, i.e. 446,02mmol/l (500mmol) NaCl and 367,93mmol/l (412mmol) urea.

In all solute concentrating means / dialysate concentrating means / CMP based separation units 310, 310A, 310B, 310C and 310D approximately 10% of the fluid was extracted via collective ducts CD via lines 311A, 311B, 311C, 311D and recombined to be returned in 302. This return fluid contains 92% of all initially extracted NaCl and 33% of all initially extracted urea. If no further utox separation was made by additional Solute purifying means / dialysate purifying means / CMP based separation units 700 the same fraction of all other utox will return. This can be too less even for 15h/d dialysis and it is recommended to implement at least one Solute purifying means / dialysate purifying means / CMP based separation unit 700 to separate NaCl and the small ions as well as bicarbonate from the larger utox. If the utox of higher molecular weight are separated by a further Solute purifying means / dialysate purifying means / CMP based separation unit 700 they can be discarded to a significantly higher amount depending on the dialysis needs. Fluid removal was calculated at its lower medical limit (anti-diuresis), if more fluid removal is needed it can be taken from one of the lines 311 or 412 outlets via a valve in a convenient linear combination. Taking out more fluid is always possible via line 412 and there is no need for fluid restrictions in the diet if patients use this system on a wearable device permanently. Overall the linear combination of all subsystems can be altered according to the medical needs using the valves without changing filters - the example is selected with emphasis on a worst case that stresses the device more than regular dialysis with its 2-31/d fluid removal. If sodium uptake is the guiding variable bicarbonate must be addressed as it is removed also while being essential. In the example also 44,381 of 25mmol/l bicarbonate is extracted, that is 1109 mmol, but most of it returns: The sodium concentration reduction factor is 146,67/154,23=0,951. So the bicarbonate return concentration will be (in worst case, probably higher) 25mmol/l*0,951=23,77mmol/l which is still a physiological value and a second criterion for maximum NaCl reduction. If 43,26 1 with 23,77mmol/l return the total bicarbonate return is 1028,49 mmol. (1109-1028,49)mmol= 80,51 mmol is the loss of bicarbonate. This is more than acceptable and requires 80,51mmol/(11,9mmol/g)=6,75g bicarbonate orally administered per day at the worst case. This still does not need technical admixture to the returning dialysate as in regular dialysis. Usually the sodium extraction is significantly smaller and so is the bicarbonate and there might be many situations where just 2g bicarbonate per day have to be replaced. The oral bicarbonate administration is subject of medical prescription when using the invention. As 6-7g can be easily handled and moreover a worst case scenario this is not critical to applicability of the invention. In general the patients full electrolyte spectrum should be carefully observed during fade in of the invention type dialysate preparation and balanced by appropriate diet. It usually can be balanced by natural nutritional habits - the unavoidable removal enforced by the invention refers to the natural process a native kidney performs and can be equilibrated by oral uptake - despite todays in-center hemodialysis.

The total amount of fluid extracted from the patient per day is 44,381/d in the example, the return is 43,261/d, thus the removal is 1,121/d. This can be used for regular dialysis but it is recommended to use wearable devices to take full advantage of the invention. This is possible: If this fluid was supplied using a wearable artificial kidney (WAK) with water chambers in it a 100kg patient would have to replace it 9 times a day using a 5kg vest, which is acceptable to a 100kg person if the water is distributed all over the vest uniformly and scales down with patient size. Smaller patients have to carry less. If no nocturnal dialysis is scheduled this means the patient would have to return to a base station every 100min for 2min to have drained 44,38 1/9=4,93 1 to the base station and fresh 43,26 1/9= 4,8 1 be flushed into the vest at every reload. The patient would lose 1,121/d this way (and should be encouraged to drink). The mean dialysate flow would be 50ml/min this way, which is sufficient for a 15h dialysis/d regime every day again, no fistula would be needed. The patients could participate in social activities and work 8h/d using a WAK and would no longer need to attend to a 3x4h regime in a dialysis center. The setup in the example is designed for a flow of 360ml/min, which is more than sufficient in this case. It will become clear later why this over-dimensioned approach is helpful.

**High or middleweight uremic toxin fractions can be separately addressed.**

Additional to the solute purifying means / dialysate purifying means / CMP based separation unit 700 (second upconcentration stage) which serves to separate NaCl from larger molecules it is possible to implement further CMP devices as further purifying means / dialysate purifying means / CMP based separation units 700A, 700B, 700C, ... (Fig. 16) to separate middle weight fractions or large molecules for addressing individual treatment to them if this is of particular interest. The transport protein fraction is of such interest. One approach could be to increase the cutoff of device 500 so that - despite to regular hemodialysis - a significant albumin and globulin fraction can pass the primary fluid generation device 500. This would mean to slightly superimpose plasmapheresis to regular dialysis. As all dialysate that is carrying these transport proteins will return there is no need of transport protein replacement. The transport protein fraction that once has passed the membrane at 500 will be highly concentrated in the last purifying means / dialysate purifying means / CMP based separation unit here 700C, even after all smaller utox have left via purifying means / dialysate purifying means / CMP based separation unit 700A and optionally 700B, so that purifying means / dialysate purifying means / CMP based separation unit 700C is entered by dialysate carrying only transport proteins as they have the highest molecular weight of all fractions. This permits to separately heat this fraction up to temperatures just below the denaturation temperature of the transport proteins or even change pH temporarily by other means if this is done buffered and reversibly. Both will unload the transport proteins to a certain extent and for a corresponding fraction of time. This happens in close proximity to the semipermeable membrane and will highly increase the chance that the protein load once released will pass the membrane by the solvent drain imposed by hydraulic pressure. The unloaded transport proteins are chilled or rebalanced in pH and returned to primary fluid generation device 500 as they leave at the concentrate or collective duct outlet of the solute purifying means / dialysate purifying means / CMP based separation unit 700C and will be returned to the body via primary fluid generation device 500. This helps to improve dialysis in terms of improving protein bound uremic toxins (PBUT) clearance significantly, will support overall PBUT transport capacity and therefore improve long term outcome from dialysis.

**Will clearance, runtime and size of the setup match requirements?**

The small elementary unit of the setup is a countercurrent multiplication device that - for best standing against pressure - in a typical embodiment but not exclusively can be designed as a round fiber in a round fiber (Fig. 6) of appropriate size and flow that diffusion across the membrane may sufficiently occur to build up the concentration profile described above according to Hargitay1951. This is typically in the sub-mm to micron range at flow speeds not significantly higher than 100 times the mean diffusion speed across the membrane. Primary urine is applied via an inlet line that comes from the primary solution generation device 500 via line 301 or a previous similar or identical unit. Pump means 600 comprising one or more pumps 610, 620 are providing the hydraulic pressure needed and valves 611, 621 may be provided to control the amount of fluid directed to descending limb DL 334 and collective duct CD 336. The fluid returns in ascending limb AL 333 after having passed a special valve at the second end 332 of tube 330 with a low cross section duct that provides a significant pressure drop compared to the pressure drop the fluids experience along 334 and 336. This is the pressure drop valve at the hairpin bend. The Valve at the second end 332 can also be omitted so that the return flow is only comprised of water that already has passed the membrane 321, because a system without return at the hairpin bend also builds up concentration due to the flow via the SPM directly which in fact is a return path at every point along the length where p can drop. This has the same effect as if there was a return valve. A facultative solution inlet is provided via an additional admixture valve 356 that allows to feed solutions of different composition but similar osmolarity like in the descending limb DL 334. Like described semipermeable filter means 320 separate DL 334 from AL 333 and semipermeable filter means 34) separate DL 334 from CD 336. An outlet orifice 337 delivers fluid of high osmolarity that was generated by the device and can be regarded as the urine in dialysis applications. It is also comprised by the invention to not implement collective duct 336 and extract the concentrate directly at the endpoint (see Hargitay1951, figure 6a, 6c). Another embodiment of the invention as shown in Fig. 5 separates the functionality within two different housings with tubes 330 and 350. The first housing comprises the concentration gradient building unit with DL 334 and AL 333, whereas the hairpin turnpoint valve may exist or not. The concentrate is extracted at valve 335 and conducted to a second functional subunit located even at a certain distance that serves as an osmotic extractor for a same or different solute that is delivered using valve 621 into an inner or outer upconcentration compartment in tube 350, wherefrom it finally can be drawn via valve 355. The outer or inner compartment is filled with the concentrate extracted from valve 335. This is helpful in particular if the two functions have to be performed at different locations due to other technical reasons. The invention comprises not only round configurations but any square, elliptic, rectangular or irregular shapes as long as the functionality of the CMP is embodied. Also the 3-dimensional alignment can be straight, curved, helical or irregular as long as the CMP can work. Also combinations of different length and therefore of different maximum upconcentration are included.

The elementary unit can be sequenced, parallelized or arranged using a combination of both. It can be used to create an environment of a desired osmolarity profile like in the kidney with its rising osmolarity from cortex to medulla tip for upconcentration using a flow-through-principle. It can also be used to create an environment of a periodic or any other type of osmolarity profile to allow for time dependent chemical reactions that are operated in a flow-through reaction pipe that depends on time alterations of osmolarity of the environment. Even pH-alterations in such a device can be realized if the osmotic active solute is a buffer substance. This means the device can be used to create a spatial and time dependent concentration of arbitrary shape within a container that can be used for chemical or pharmaceutical reactions.

With focus on medical dialysis the numerical simulation described above was also used to find out what technical sizes would be required to realize a 360ml/min input flow (not clearance). This value can be regarded as typical for dialysis devices in a 3 by 4 hours regime as well as it would be sufficient to build the solute concentrating means / dialysate concentrating means / CMP based separation units 310 just one time per device and use it subsequent in time to allow for smaller total outlines when it comes to wearable dialysis applications and transportability. This would be a balanced tradeoff between weight and operation time. In this applications it is important to keep the full filter and cryo unit below 15kg to have it still transportable in a base station that exchanges dialysate carried within a low weight body worn dialysis west or trousers. Otherwise it cannot be transported as hand luggage in an aircraft or car.

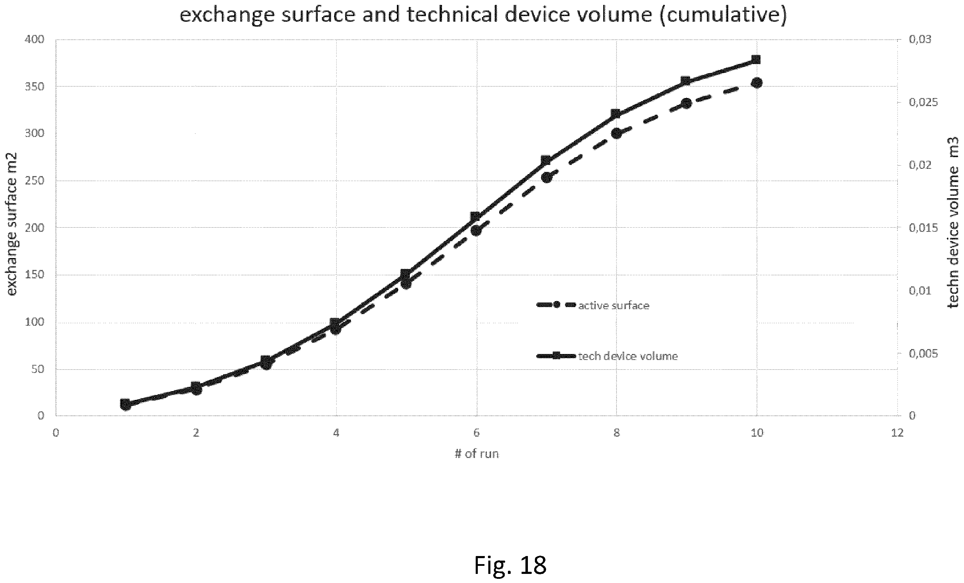

Important for total size is the grade of parallelization and the runtimes per purification unit, in particular how often solute concentrating means / dialysate concentrating means / CMP based separation unit 310 is implemented and how long a single run takes. The runtimes required for the example result from the runtime of the slowest stage of the parallel execution and are shown in fig. 18. It should be understood that the early execute faster. However, the total runtime for successive runs on only one filter unit would then have to be calculated as the sum of all runtimes - only if all stages run on separate hardware (multi filter system) the slowest separation unit alone is decisive.

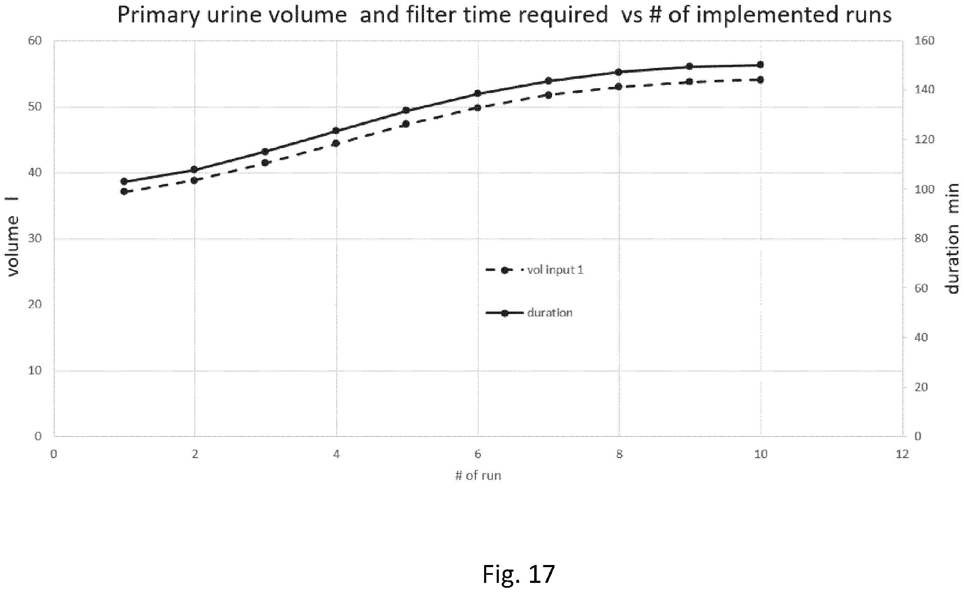

In Fig. 17 also the total volume of primary urine per day required for the example is visible - e.g. from a portable vest - that has to be provided to remove the amount of solutes prescribed when the system runs up to a certain pass. Fig. 17 demonstrates the more runs required, the more primary urine has to be provided which will soon become impractical. Therefore, for wearable solutions, an attempt should be made to implement as few passes as possible.

With respect to the example reading the curves of Fig. 17 clarifies how the number of purification units of a given property are chosen. If a volume of 451 primary urine is at the practicability limit not more than four solute concentrating means / dialysate concentrating means / CMP based separation units 310 with 360ml/min clearance should be implemented. Otherwise the vest refilling intervals or the amount of water to be worn by the patient would become impractical. If these 4 units take a duration of (105+110+115+125)min= appr. 7.5h, additionally another 3h are needed for the cryo purification, it is apparent that the setup will be busy 10h/d to clear the 451 of the example in successive operation. Even for a non-parallelized system this is shorter than dialysis time per day using a WAK so the invention matches the requirement of sufficient flow supply. This is not a must and can be quicker using parallelized systems but saves weight and assures transportability. This seems to be convenient and handy if the machine works software controlled and unattended. Certain additional service intervals will be mandatory so that the total operation of a setup according to the invention can be handled within the awake period and provides sufficient flow.

The technical filter volume was determined in the example from the pure volume of the exchange chambers in DL, AL, CD - volume requirements for wall thicknesses, distribution volumes, pipe guides, valves, pumps, housings and other technical equipment were not taken into account as they depend on the technical materials and technology selected finally. The exchange area refers to the total exchange areas between all upper and lower limbs necessary for the clearance required in the example. It was determined within the simulation how many individual modules like depicted in Fig. 6 are required to achieve the performance data of the medical prescription. With respect to the simplification described their total volume and total exchange area is given in Fig. 18.

It can be seen from the two curves of Fig. 18 that the additional volume requirement does not increase equally for all the selected numbers of passages. As one would also expect, it is advantageous to select only as many separation units until a linear combination of the outlets 411 and 701 in Figs. 1, 2 or 16 can fulfil the sodium and urea removal prescription of dialysis as well as the practicability limit, see Fig. 17, which is 4 in the example. The simulation yields an exchange surface of 91,98m2 for the cumulative surface if four filter unit are used simultaneously to be quicker. If only one filter unit at a time is used it must be built with the largest surface necessary, that is the difference of run 4-run3, (91,98-54,8)m2= 37,18m2. This means the active surface size of the unit in Fig. 7 shrinks to 40% if subsequent filter use is made. In this region the last sequence determines the exchange surface of the filter unit as it has the highest surface requirements. The purely technical filter volume in parallel operation is 7,361, if the operation is subsequent the filter volume is only 2,97 1, again 40%. This means to build a complete unit with 360ml/min flow input it is necessary to have at least an active SPM surface of 37,18m2 for dialysis. If only one purification unit is used sequentially it must be comprised of 3.720.000 subunits of Fig. 6, each having a length of 10mm, in total 37,2km. If in a general case different solutions for upconcentration and for clearing are intended, SMP2 with approximately the same surface is needed but could be multiplexed also if a configuration like in Fig. 5 is used - this will save the SMP2 also in this general case. All numbers indicate that it is realistic to pack all the CMP purification units into a cabin suitcase for medical dialysis purposes.

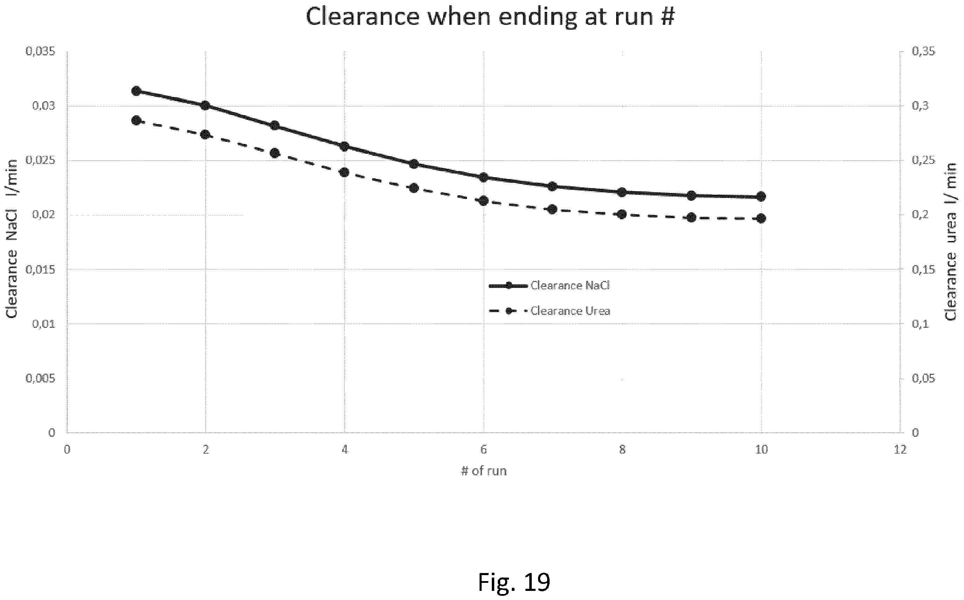

In parallel operation the clearance for NaCl and urea that can be achieved with the setup according to the invention in the configuration of table 2 (high NaCl extraction, anti-diuresis) is depicted in Fig. 19. It can be seen that using four purification stages (#run) a urea clearance of 239ml/min compared to a sodium clearance of 26,3ml/min can be achieved, both out of 360ml/min input flow. Usually sodium and urea clearance do not significantly differ on dialysis filters. This ratio is a good approach to the factor of 8.5 calculated above and marks an antidiuresis range endpoint achievable with the parameters of table 2. If higher separation or less urine quantity is desired the technical outline of the micro-unit of Fig. 6 (length, sizes, membrane type, pressure) must be modified to also cover other parameters like they may occur outside medical dialysis. The example however was designed to demonstrate that the invention is able to perform medical dialysate reclamation using technically available fibers from the reverse osmosis field with sufficient return of all substances usually admixed in regular dialysate preparation to water. In this sense - but only in this sense - the invention describes a technical kidney that can perform decentral medical dialysis when combined with a WAK and a base station. To technically realize the upconcentration function of the kidney therefore is no longer impossible using the approach of combination of CMP in filter devices and cryo-purification. Moreover it can also be used for chemical and pharmaceutical concentration purposes far beyond the scope of medical dialysis.

**A wearable dialysis device**

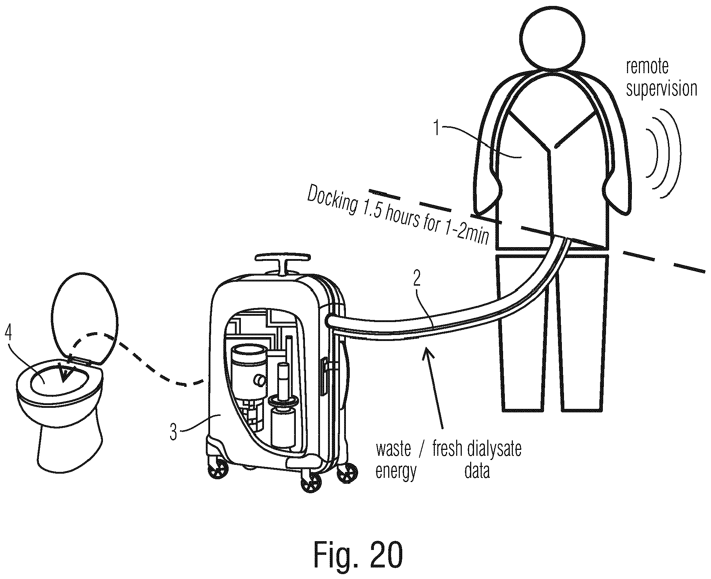

Fig. 20 is an embodiment for a wearable dialysis device comprising a wearable part 1, which can be a vest or the like, and a portable part 3, which can be trolley or the like. Connection lines 2 are connecting parts 1 and 2, optionally in addition to a wireless communication. The urine can be discharged via discharge means 4, which can be a standard toilette or the like. The size and technical filter volume from the example allows to implement the solute concentrating means / dialysate concentrating means / CMP based separation units 310 and solute purifying means / dialysate purifying means / CMP based separation units 700 together with the water separation means 400 / cryo-purifier / cryo-based dialysate regeneration unit 410 into a transportable trolley of approximately 15kg once all components are fully developed. This is small enough to serve as a dialysate base for a wearable dialysis device like a vest or trousers worn during the day. The base station is located decentral at work, at home or during travel without additional external support like water, concentrate or bicarbonate except electricity. The 100kg patient would connect to the base station every 90-100 min for 2 min to flush and receive 4-51 of dialysate at a convenient temperature during 15h of the day.

**Claims**

1. Apparatus for generating dialysate for dialysis comprising

   dialysate purifying means (300) having

   a dialysate inlet (301) being adapted and arranged for receiving dialysate to be purified,
   at least one dialysate concentrating means (310) being adapted and arranged for concentrating substances to be maintained in the dialysate, the dialysate concentrating means (310) having a concentrate outlet (311) and a diluate outlet (312),
   and
   a dialysate outlet (302) being adapted and arranged for feeding back the purified dialysate, wherein the concentrate outlet (311) of the at least one dialysate concentrating means (310) is connected to the dialysate outlet (302),

   **characterized by** at least one water separating means (400) being adapted and arranged for separating water, the at least one water separating means (400) having an inlet (414) and a water outlet (412), wherein the inlet (414) of the at least one water separating means (400) is connected to the diluate outlet (312) of the at least one dialysate concentrating means (310) and the water outlet (412) of the at least one water separating means (400) is connected to the dialysate outlet (302).

2. Apparatus according to claim 1, further comprising at least one dialysate purifying means (700) being adapted and arranged for removing substances from the dialysate, the dialysate purifying means (700) having a concentrate outlet (701) and a diluate outlet (702), wherein the diluate outlet (702) of the at least one dialysate purifying means (700) is connected to the dialysate outlet (302).

3. Apparatus according to any of claims 1 to 2, wherein the at least one water separating means (400) comprises a cryo-purifier (410).

4. Apparatus according to any of claims 1 to 2, wherein the at least one water separating means (400) comprises means for removing urea from aqueous solutions and/or urease means and/or at least one aquaporin membrane.

5. Apparatus according to any of the preceding claims, wherein the at least one dialysate concentrating means (310) and/or the at least one dialysate purifying means (700) are adapted and arranged according to the countercurrent multiplication principle (CMP).

6. Apparatus according to any of the preceding claims, wherein the at least one dialysate concentrating means (310) and/or the at least one dialysate purifying means (700) comprise a semipermeable filter means (320), a first flow path (333) at a first side (323) of the semipermeable filter means (320), and a second flow path (334) at a second side (324) of the semipermeable filter means (320), being opposite to the first side (323) of the semipermeable filter means (320).

7. Apparatus according to any of the preceding claims, wherein the at least one dialysate concentrating means (310) and/or the at least one dialysate purifying means (700) comprise pump means (600) being adapted and arranged for applying pressure to the at least one dialysate concentrating means (310) and/or the at least one dialysate purifying means (700).

8. Apparatus according to any of the preceding claims, wherein the at least one dialysate concentrating means (310) and/or the at least one dialysate purifying means (700) are adapted and arranged to provide several successive and/or concurrent stages.

9. Apparatus according to any of the preceding claims, wherein the at least one dialysate concentrating means (310) are being adapted and arranged to provide at least one cutoff for electrolytes and/or bicarbonates, and/or wherein the at least one dialysate purifying means (700) are being adapted and arranged to provide at least one cutoff for uremic toxins, and/or further comprising at least one additional dialysate purifying means (700B) being adapted and arranged to provide at least one cutoff for transport proteins, the at least one additional dialysate purifying means (700B) preferably comprising heating means (780) and/or a pH modification means.

10. Hydraulic pressure driven solute concentrating means (310) being adapted and arranged for concentrating substances to be maintained as solute in the solvent where the solute is solved, the solute concentrating means (310) having a concentrate outlet (311) and a diluate outlet (312), and a first semipermeable filter means (320) being adapted and arranged to separate first solutes from the solvent, the semipermeable filter means (320) being located between a first flow path (333) at a first side (323) of the semipermeable filter means (320), and a second flow path (334) at a second side (324) of the semipermeable filter means (320), being opposite to the first side (323) of the semipermeable filter means (320), **characterized in that** the first flow path (333) and the second flow path (334) are adapted and arranged to have at least one cross section wherein the first flow path (333) surrounds the second flow path (334) or the second flow path (334) surrounds the first flow path (333).

11. Hydraulic pressure driven solute concentrating means (310) according to claim 10, wherein the semipermeable filter means (320) are adapted and arranged to have a tubular form, wherein the first flow path (333) is arranged inside the tubular form of the semipermeable filter means (320) and the second flow path (334) is arranged outside the tubular form of the semipermeable filter means (320), or

    wherein the second flow path (334) is arranged inside the tubular form of the semipermeable filter means (320) and the first flow path (333) is arranged outside the tubular form of the semipermeable filter means (320) .

12. Hydraulic pressure driven solute concentrating means (310) according to any of claims 10 to 11, further comprising second semipermeable filter means (340) being adapted and arranged to separate a third flow path (336) from the first flow path (333) or the second flow path (334) or

    being adapted and arranged to separate a third flow path (336) from a flow path connected to the output of the first flow path (333) or the second flow path (334).

13. Hydraulic pressure driven solute concentrating means (310) according to claim 12, wherein the second semipermeable filter means (340) is adapted and arranged to surround the first semipermeable filter means (320) such that the second semipermeable filter means (340) is arranged between the third flow path (336) and the second flow path (334) and the first semipermeable filter means (320) is arranged between the second flow path (334) and the first flow path (333).

14. Hydraulic pressure driven solute concentrating means (310) according to claim 12, wherein the second semipermeable filter means (340) and the first semipermeable filter means (320) are adapted and arranged in separate housings.

15. Apparatus according to any of claims 1 to 9, wherein the at least one dialysate concentrating means (310) and/or several dialysate concentrating means (310) are adapted and arranged according to a hydraulic pressure driven solute concentrating means according to any of claims 10 to 14.

Fig. 1

Fig. 2

Fig. 3

Fig. 4

Fig. 5

EP 4 335 468 A1

Fig. 6

Fig. 7

$u_{10}$=inflow velocity at CD, DL
$u_{20}$=outflow velocity at AL
$c_L$ =concentration at L (hairpin tip or CD output)
$c_{ij}$ = concentration
i  =1 refers to DL or CD; 2 for AL
j  =location ref  0 for x=0, L for x=L
L = length of the limbs
p = pressure
a = width of DL, AL (height=1)
b = width of CD   (height=1)
$v_x$ , $v_L$ = factor of concentration at x, at L
        =$c_{1x}/c_{10}$, $c_{1L}/c_{10}$
V = Volume
Q = flow ; Q=a*u(*h)=V/t=Q
b is chosen such that u in CD, DL identical
$\vartheta$  = b/(a+b)
$\xi = \dfrac{1}{v_L}\vartheta$
P = hydraulic/osmotic pressure at x=0

$\gamma$ = SPM-permeability
$= [\dfrac{m^3}{s} \dfrac{1}{m^2} \dfrac{m^2}{N}] = [\dfrac{m^3}{s} \dfrac{1}{m^2} \dfrac{m\,s^2}{kg\,m}]$
$= \dfrac{m^2 s}{kg} = 10 \dfrac{cm^2 s}{g}$

Fig. 8

$t_0$

$p$

solvent : $H_2O$
solute: any molecule not
permeating SPM

$c_0$   $v_0$   $\pi_0$

— — — — — — SMP

$c_0$   $v_0$   $\pi_0$

$t_1$

$p$

water transistion $\Delta v$

$c_1 > c_0$  $v_1 = v_0 - \Delta v$
$\pi_1$

— — — — — — SMP

$c_2 < c_0$  $v_2 = v_0 + \Delta v$
$\pi_2$

Fig. 9

1.

off

$\Delta p = 0$
$t = 0$
valve closed

p=0; D40

p=0; D40

diffusion
time

2.

on

$\Delta p = p$
$t = 7s$
valve opens

p=p; D50

p=0; D30

3.

on

$\Delta p = p$
$t = 8s$
valve closes

D40

p=p; D50

p=0; D30

D50

diffusion
time

Fig. 10

Fig. 11

EP 4 335 468 A1

**7.**

on

| D40 | D45 | D47,5 | p=p; D50 | | D55 |
|-----|-----|-------|----------|--|-----|
| D27,5 | p=0; D30 | | D35 | D45 | D65 |

Δp=p
t=24s
valve closes

diffusion
time

**8.**

on

| D43,7 | D47,5 | D48,8 | p=p; D50 | D52,5 | D57,5 | D70 |
|-------|-------|-------|----------|-------|-------|-----|
| D23,7 | D27,5 | D28,8 | p=0; D30 | D32,5 | D37,5 | D50 |

Δp=p
t=31s
valve opens

Dmean    D33,7    D37,5    D38,8    D40    D42,5    D47,5    D60

Fig. 12

Fig. 13

$$C_X = \frac{C_0}{1 - \frac{\gamma p}{a\, u_0} x}$$

Fig. 14  (prior art)

[NaCl] along x after 400 calc steps in top and bottom compartment (DL and CD, AL)

Fig. 15

Fig. 16

EP 4 335 468 A1

Primary urine volume and filter time required vs # of implemented runs

Fig. 17

Fig. 18

Fig. 19

Fig. 20

Fig. 21

Fig. 22

Fig. 23

EP 4 335 468 A1

Europäisches
Patentamt

European
Patent Office

Office européen
des brevets

# EUROPEAN SEARCH REPORT

**Application Number**

EP 22 19 4710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/114595 A1 (WALLENAS ANDERS [SE] ET AL) 7 May 2009 (2009-05-07) | 1,2,4-9 | INV.<br>A61M1/16<br>A61M1/28 |
| Y | * paragraph [0001] *<br>* paragraph [0041]; figure 1 *<br>* paragraph [0045] – paragraph [0047] *<br>* paragraph [0048] *<br>* paragraphs [0100] – [0112]; figures 14-15 * | 3 | |
| Y | US 2019/224401 A1 (GOLDAU RAINER [DE]) 25 July 2019 (2019-07-25)<br>* figure 2 *<br>* paragraph [0141] *<br>* paragraph [0241] *<br>* the whole document * | 3 | |

| | | |
|---|---|---|
| | | TECHNICAL FIELDS SEARCHED (IPC) |
| | | A61M |

The present search report has been drawn up for all claims

| Place of search | Date of completion of the search | Examiner |
|---|---|---|
| The Hague | 17 April 2023 | Ugarte, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................
& : member of the same patent family, corresponding document

EPO FORM 1503 03.82 (P04C01)

Europäisches
Patentamt

European
Patent Office

Office européen
des brevets

**Application Number**

**EP 22 19 4710**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

[X] None of the further search fees have been paid within the fixed time limit. The present European search report has been drawn up for those parts of the European patent application which relate to the invention first mentioned in the claims, namely claims:

1-9

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

EP 22 19 4710

The Search Division considers that the present European patent application does not comply with the requirements of unity of invention and relates to several inventions or groups of inventions, namely:

**1. claims: 1-9**

Apparatus for generating dialysate for dialysis comprising dialysate purifying means havinga dialysate inlet being adapted and arranged for receiving dialysate to be purified, at least one dialysate concentrating means being adapted and arranged for concentrating substances to be maintained in the dialysate, the dialysate concentrating means having a concentrate outlet and a diluate outlet, anda dialysate outlet being adapted and arranged for feeding back the purified dialysate, wherein the concentrate outlet of the at least one dialysate concentrating means is connected to the dialysate outlet, characterized byat least one water separating means being adapted and arranged for separating water, the at least one water separating means having an inlet and a water outlet, wherein the inlet of the at least one water separating means is connected to the diluate outlet of the at least one dialysate concentrating means and the water outlet of the at least one water separating means is connected to the dialysate outlet.

---

**2. claims: 10-15**

Hydraulic pressure driven solute concentrating means being adapted and arranged for concentrating substances to be maintained as solute in the solvent where the solute is solved, the solute concentrating means having a concentrate outlet and a diluate outlet, and a first semipermeable filter means being adapted and arranged to separate first solutes from the solvent, the semipermeable filter means being located between a first flow path at a first side of the semipermeable filter means, and a second flow path at a second side of the semipermeable filter means, being opposite to the first side of the semipermeable filter means, characterized in thatthe first flow path and the second flow path are adapted and arranged to have at least one cross section wherein the first flow path surrounds the second flow path or the second flow path surrounds the first flow path.

---

## ANNEX TO THE EUROPEAN SEARCH REPORT
## ON EUROPEAN PATENT APPLICATION NO.

EP 22 19 4710

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2023

| Patent document cited in search report | | Publication date | Patent family member(s) | | Publication date |
|---|---|---|---|---|---|
| US 2009114595 | A1 | 07-05-2009 | AU | 2006214829 A1 | 24-08-2006 |
| | | | CA | 2598486 A1 | 24-08-2006 |
| | | | CN | 101155607 A | 02-04-2008 |
| | | | EP | 1933898 A2 | 25-06-2008 |
| | | | JP | 4594990 B2 | 08-12-2010 |
| | | | JP | 2008529715 A | 07-08-2008 |
| | | | US | 2009114595 A1 | 07-05-2009 |
| | | | WO | 2006088419 A2 | 24-08-2006 |
| US 2019224401 | A1 | 25-07-2019 | AU | 2017300847 A1 | 13-12-2018 |
| | | | CA | 3025598 A1 | 25-01-2018 |
| | | | CN | 109414540 A | 01-03-2019 |
| | | | DK | 3487555 T3 | 13-07-2020 |
| | | | EP | 3272375 A1 | 24-01-2018 |
| | | | EP | 3487555 A1 | 29-05-2019 |
| | | | ES | 2791490 T3 | 04-11-2020 |
| | | | JP | 6970123 B2 | 24-11-2021 |
| | | | JP | 2019527074 A | 26-09-2019 |
| | | | US | 2019224401 A1 | 25-07-2019 |
| | | | WO | 2018015571 A1 | 25-01-2018 |
| | | | ZA | 201900959 B | 30-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3487555 B1
- US 2003187380 A1
- EP 2586473 A1

**Non-patent literature cited in the description**

- **E. WEINBERG et al.** Concept and computational design for a bioartificial nephron-on-a-chip. *The international journal of artificial organs,* June 2008, vol. 31 (6), 508-514
- Handbook of Membrane separations. CRC, 2009
- **HARGITAY ; KUHN.** *Hargitay,* 1951
- **NIERE ; HARGITAY B ; KUHN W.** *Zeitschrift fur Elektrochemie,* August 1951, vol. 55 (6), 539
- **HARGITAY B ; KUHN W.** The multiplication principle as the basis for concentrating urine in the kidney. *J Am Soc Nephrol,* 2001, vol. 12, 1566
- **DURATONF ; COHENG et al.** *JAmSocNephrol,* 2012, vol. 23, 1258